# DEMANDE DE BREVET EUROPEEN

(11) **EP 1 159 957 A2**
(43) Date de publication de la demande: **05.12.2001**
(21) Numéro de dépôt: 01118964.4
(22) Date de dépôt: 02.12.1997
(51) Int. Cl.: A61K 9/22

(54) **Dispositif d'administration locale de formulations solides ou semi-solides, formulations retard pour administration parentérale et procédé de préparation**

(30) Priorité: 02.12.1996 FR 9614755
(62) Demande divisionnaire de: 97948983.8
(71) Demandeur: SOCIETE DE CONSEILS DE RECHERCHES ET D'APPLICATIONS SCIENTIFIQUES (S.C.R.A.S.), 75016 Paris (FR)
(72) Inventeur: Cherif Cheikh, Roland, 92130 Issy Les Moulineaux (FR)
(74) Mandataire: Bernasconi, Jean Raymond

(57) **Abrégé**

L'invention a pour objet l'utilisation, pour l'implantation ou l'insertion d'une formulation solide (1, 9) ou semi-solide (18), destinée à être mise en place en un site de dépôt précis (T) d'un organisme, contenant au moins un principe actif, ladite formulation étant de consistance solide ou semi-solide telle qu'elle puisse persister pendant une certaine durée dans le site, et contenant une dose de principe actif limitée pour un traitement dans une zone ciblée de l'organisme, d'un dispositif comprenant une partie placée à l'intérieur du corps du patient avec des moyens de conditionnement de la forme solide sou semi-solide, des moyens de positionnement permettant d'amener ces moyens de conditionnement jusqu'au site de dépôt, des moyens d'injection ou d'insertion à ce site de dépôt, et des moyens de retrait après injection ou insertion, et une partie laissée à l'extérieur avec des moyens d'activation des fonctions du dispositif. L'invention a également pour objet une formulation retard solide pour administration parentérale comprenant un mélange homogène d'un principe actif à l'état non dispersé et d'un excipient biocompatible biodégradable, dans laquelle la quantité de principe actif est d'au moins 50% en poids.

## Description

La présente invention a pour objet un procédé thérapeutique pour permettre le traitement ciblé de formulations galéniques non liquides ainsi que la préparation et le dispositif conçu pour la mise en oeuvre du procédé.

On connaît les avantages de l'utilisation d'un traitement ou d'une administration locale lorsque le principe actif (PA) est ainsi préférentiellement orienté vers son site d'action. Il est prouvé, d'autre part, que l'administration orale ou parentérale d'un médicament et sa diffusion systémique peuvent, dans certains cas, ne cas, pas donner un résultat satisfaisant. Par ailleurs, même dans le cas où l'on vise un traitement général ou systémique, notamment dans le cas des formulations retard, il est intéressant d'insérer la formulation dans un site adapté.

En dehors de l'amélioration de l'efficacité locale, le traitement local par rapport à un traitement général permet avant tout de diminuer les doses et les effets secondaires notamment liés au P.A. dans des sites de l'organisme où sa présence est soit inutile soit nuisible.

L'administration locale d'un médicament permet, donc, d'améliorer l'index thérapeutique du produit tout en diminuant le cas échéant sa toxicité générale et les risques d'effets systémiques.

Les formes topiques cutanées, oculaires, naso-sinusiennes, pulmonaires ou encore gastriques ou rectales ont été les premières formes non parentérales à utiliser l'administration locale. Lorsque le site de dépôt de la formulation est plus difficilement accessible ou nécessite une forme invasive et lorsque le traitement doit être répété, ou plus encore, chronique, même si l'avantage d'un ciblage est connu, dans la pratique, son utilisation se heurte à la difficulté voire à l'inconfort d'un geste thérapeutique répété.

On connaît, d'autre part, les avantages de l'utilisation d'une formulation prolongée ou retard qui permet, en une seule administration, de donner au malade son médicament pour plusieurs jours, plusieurs semaines ou plusieurs mois.

Cette forme retard améliore la compliance puisque l'observance du traitement ne dépend plus du malade ou du personnel soignant mais de la préparation. Cet effet prolongé améliore donc le confort du patient qui n'est plus astreint à son traitement, et qui reçoit ainsi une dose régulière en permanence et non variable en fonction des prises de médication.

Le développement des formes retards a conduit les spécialistes à considérer leurs utilisations locales notamment dans le cas mentionné précédemment où le site de dépôt est plus difficilement accessible. La forme retard évite, alors, d'avoir à répéter les administrations ou, plus encore, le geste chirurgical. On peut, alors espérer des concentrations locales importantes de médicament sur une période prolongée sans doses systémiques importantes, donc, avec moins d'effets secondaires. Cette solution est plus particulièrement utile pour les produits rapidement métabolisés ou ayant une demi-vie courte lorsqu'ils sont administrés par voie systémique.

A l'intérieur de l'organisme, on envisage ainsi des traitements ciblés et prolongés tel que les injections intra- ou péri-articulaires de corticoïdes retard. Les cancers et notamment les tumeurs solides sont des candidats de choix pour ces formes locales qui permettent de diminuer les doses totales injectées de composé cytotoxique ou antinéoplasique, tout en augmentant la concentration dans la zone tumorale à traiter. Ceci est donc susceptible d'éviter les graves effets secondaires de ce type de traitement.

Matrix Pharmaceutical propose une préparation retard à base de collagène qui pourra être injectée en intra-tumorale (IntraDose CDDP-Cisplatin). Cette formulation est administrée dans les cancers ou les lésions cutanées à l'aide d'une seringue de 3 cc et éventuellement une aiguille de biopsie pour les zones moins accessibles. Sous un volume liquide visqueux pouvant atteindre 2ml, elle est donc limitée aux sites d'abord relativement facile (périphérique) ou aux traitements post-chirurgicaux.

On peut aussi citer le brevet MITSUI (FR 2 497 661; JP 562 737) qui décrit une forme polylactide-polyglycolide (PLGA) en bâtonnet ou en aiguille pour activité locale, permettant son implantation directe dans une zone ou un organe à l'intérieur de l'organisme, et par exemple une zone tumorale avant ou après exérèse.

La forme Gliadel (Guilford) décrit pour sa part une formulation à base de polyanhydride en forme d'hostie contenant de la Carmustine et qui peut, par exemple, être déposée au moment de la chirurgie au niveau d'une tumeur cérébrale (glioblastome).

Dans l'état actuel de la technologie médicale, ces traitements ciblés à l'intérieur de l'organisme sont encore souvent liés à des interventions chirurgicales lourdes. Ils bénéficient de l'effet prolongé de la formulation mais ne peuvent pas être facilement répétés.

On pratique, aussi, des interventions de chimio-embolisation qui consistent à injecter, dans des vaisseaux, des suspensions (microsphères), des gels ou des colles avec leur solvant, qui pourront obstruer un trajet vasculaire nourricier et libérer un P.A. au niveau d'une tumeur. L'occlusion est obtenue par dépôt après départ du véhicule d'injection. Cette technique utilise les cathéters d'angioplastie percutanée transluminale pour introduire le fluide dans le vaisseau.

L'utilisation locale des formes retard est également envisagée dans certaines cavités corporelles et dans des sites plus accessibles de l'organisme.

Le système ®Ocusert (Alza) est un insert oculaire flexible et ovale qui constitue un dispositif réservoir retard comportant une membrane en copolymère éthylène-acétate de vinyle et qui peut contenir, par exemple, de la pilocarpine.

Ce dispositif est placé dans le cul de sac conjonctival et libère son produit selon un profil d'ordre zéro. La forme retard permet de diminuer significativement la dose nécessaire pour le même effet sur la pression intra-oculaire. L'efficacité thérapeutique de la pilocarpine dans le traitement du glaucome est ainsi 8 à 10 fois meilleure grâce à l'utilisation d'une forme retard comparée aux gouttes en local.

Le brevet américain n° 3.545.439 dont le contenu est incorporé par référence décrit une forme retard intra-vaginale constituée d'un anneau fabriqué avec un élastomère de silicone et qui libère un médicament pendant plusieurs semaines.

Dans ce cas, l'administration retard locale sur la muqueuse vaginale permet aussi, selon le P.A., d'obtenir un effet général (contraception).

Le dispositif médical décrit par Bukh Meditee (demande de brevet internationale PCT n° WO89/03232 dont le contenu est incorporé par référence) permet l'introduction dans une cavité corporelle d'une forme retard matricielle faite d'une substance faiblement pénétrable par l'eau et contenant un P.A.

La forme retard associée au dispositif délivre ainsi le P.A. au niveau local et pendant la durée de l'insertion du dit dispositif. II décrit, par exemple, un cathéter pour l'urètre débouchant dans la vessie associé à une forme retard antibiotique susceptible de prévenir les infections des voies. urinaires.

Pour des formes liquides de grand volume, certains procédés existants d'injection locale pourraient être utilisés. A partir des techniques intra-urétrales, C.R.BARD, par exemple, a développé une formulation (Transurethal delivery Kit) qui est une seringue contenant une solution de collagène dans du glutaraldéhyde qui peut être facilement injectée en sous-musocal sous des volumes de 2,5 à 7,5 ml qui constituent des implants sans principe actif dans le cadre d'une plastie contre l'incontinence.

Le développement des systèmes vasculaires intra-luminaux a conduit à la réalisation de cathéters permettant de libérer un P.A. au niveau local à leur extrémité. Contrairement au cathéter simplement ouvert pour libérer le fluide, l'administration locale peut être obtenue avec un cathéter à double ballonnet ou poreux à multiples perforations. Cette solution locale est toutefois limitée par le temps d'insertion du cathéter. La pression de la solution nécessaire pour pénétrer la paroi pose également un problème de tolérance.

Pour les solutions liquides, une véritable injection locale peut être obtenue dans la paroi à l'aide d'un système d'injection associé à un ballonnet (Interventional Technologies) ou d'un cathéter à aiguille rétractable (Bavarian Medical Technologies). L'application du médicament n'est toutefois pas beaucoup plus prolongée avec ces formes liquides immédiates.

Une partie du dispositif peut parfois être laissée en local et donc être associée à une forme retard. C'est le cas des "stents" utilisés, par exemple en angioplastie, pour éviter la resténose, qui peuvent être recouverts d'une couche contenant un principe actif parfois avec un effet retard. Deux problèmes essentiels se posent alors, le premier est l'adéquation du médicament relargué au procédé spécifique de "coating". Le second est la limitation de la dose totale par l'espace et la surface offerte par le stent.

Avec l'héparine, par exemple, certains travaux mentionnent l'importance du traitement local pour éviter les effets secondaires systémiques. Selon ces études, l'héparine inhibe la prolifération des cellules musculaires lisses après lésion endothéliale. Son administration systémique, sous forme retard sous-cutanée ou locale adjacente externe au vaisseau, conduit toujours à une diminution de la prolifération néointimale mais la forme locale est la seule à ne pas entraîner de perturbations systémiques de la coagulation.

On pourrait, encore, citer les pompes osmotiques qui sont utilisées pour valider les administrations locales prolongées avec comme inconvénient majeur leur implantation chirurgicale. Pour cette raison, elles ne sont pas actuellement utilisées chez l'homme.

Tous ces exemples montrent bien l'intérêt et les avantages apportés par un traitement ciblé surtout s'il peut être prolongé.

Ces solutions techniques présentent toutefois, toutes, certains inconvénients au nombre desquels les plus importants sont l'absence de polyvalence de la solution retenue, l'association à un dispositif spécifique qui reste totalement ou partiellement inséré sur la durée de relargage du médicament et, enfin, la limite du volume injectable, donc, de la dose de P.A.

Chacune de ces solutions permet de traiter seulement un ou plusieurs cas particuliers dans un site bien précis de l'organisme.

La vectorisation par administration locale est parfois qualifiée de première génération par rapport aux formulations "prodrugs" et vecteurs (liposomes...) dit de seconde génération ou aux systèmes de reconnaissance macromoléculaire ou activation "site spécifique" dit de troisième génération. Ces solutions, plus encore que les techniques actuelles d'administration locales, sont toutefois très spécifiques, pas toujours applicables et parfois peu précises.

L'invention a pour but de proposer un procédé remédiant aux inconvénients majeurs actuels de l'administration locale ou vectorisation par les techniques de chirurgie endoscopiques flexible (fibroscopie) ou rigide (endoscope) et de radiologie interventionnelle (cathéter actif ou non).

Les formulations non dispersées solides et semi-solides présentent l'avantage d'offrir un volume minimal pour une quantité de P.A. correspondant à une dose de traitement. Les formes retards solides et semi-solides peuvent, ainsi, permettre plusieurs jours de traitement dans un volume. de quelques micro-litres.

L'administration locale d'un traitement permet de diminuer de façon importante la dose thérapeutique totale pour un même effet.

La combinaison d'une forme retard solide ou semi-solide et d'une administration locale conduit donc à la réalisation de micro-dosages particulièrement adaptés à un dépôt local à intervalles de temps espacés.

Le développement actuel des technologiques d'imagerie, d'optiques et de micro-mécaniques appliquées au médical dans le domaine de l'instrumentation intra-vasculaire ou cavitaire et de la chirurgie à invasion minimale a conduit à la conception d'outils de plus en plus fins et de plus en plus précis permettant d'intervenir localement très profondément dans l'organisme avec un traumatisme minimal et donc de multiplier les sites accessibles.

L'invention propose donc un procédé, un dispositif et une formulation adaptés au progrès et à la miniaturisation des technologies pharmaceutiques et médicales.

Le procédé visé par l'invention pour l'implantation ou l'insertion d'un principe actif contenu dans une formulation solide ou semi-solide en un site de dépôt précis de l'organisme est caractérisé en ce qu'il consiste à obtenir la formulation solide ou semi-solide, à charger cette formulation dans un dispositif pouvant être actionné de l'extérieur de l'organisme, à amener le dit dispositif au dit site de dépôt à l'intérieur d'outils classiques d'intervention à ce niveau, et à insérer ou implanter la dite formulation en actionnant le dit dispositif.

Selon d'autres particularités de l'invention :
- ledit site de dépôt n'est pas accessible avec une seringue ou un trocart hypodermique classique,
- ladite formulation solide ou semi-solide a une forme fine et allongée une fois chargée dans ledit dispositif,
- ledit dispositif est fin et allongé pour pouvoir circuler dans les dits outils classiques d'intervention,
- la dite formulation solide ou semi-solide est une formulation retard,
- la dite forme fine et allongée offre un rapport minimum longueur sur diamètre de 10,
- ledit dispositif est le contenant de la dite formulation juste adaptée à la dite forme,
- la dite forme et le dit dispositif sont cylindriques,
- la dite implantation a lieu dans un tissu, dans une muqueuse ou une paroi interne de l'organisme par voie cavitaire,
- la dite implantation a lieu dans un tissu, dans une muqueuse ou une paroi interne de l'organisme par voie vasculaire, artérielle ou veineuse,
- la dite implantation a lieu dans un tissu, dans une tumeur ou une zone pathogène par voie chirurgicale,
- la dite insertion a lieu dans une cavité corporelle ou dans un organe par voie cavitaire,
- la dite insertion a lieu dans une cavité corporelle ou dans un organe ou un tissu par voie invasive ou chirurgicale,
- ledit principe actif est un anti-inflammatoire,
- ledit principe actif est un peptide ou un analogue de peptide,
- ledit principe actif est un produit anticancéreux,
- ledit principe actif est un mélange de 2 ou plusieurs principes actifs.

L'invention a aussi pour objet :
- une méthode de traitement thérapeutique dans laquelle un principe actif dans une formulation solide ou semi-solide est inséré dans une cavité corporelle de façon que le dit principe actif soit libéré dans les fluides à la surface de la dite cavité et puisse agir localement-vers les sites de drainage des dits fluides corporels,
- une méthode de traitement thérapeutique dans lequel un principe actif dans une formulation solide ou semi-solide est implanté dans une muqueuse ou un tissu sécrétoire interne de l'organisme de façon que le dit principe actif soit libéré et excrété avec les fluides naturels et puisse agir localement ou vers les sites de drainage,
- une méthode de traitement dans laquelle le dit principe actif a une action locale et/ou systémique à partir du site de dépôt de l'implant,
- une méthode de traitement thérapeutique des pathologies ORL dans laquelle le principe actif dans une formulation solide ou semi-solide est introduit dans une cavité de la face ou dans la muqueuse qui la recouvre,
- une méthode de traitement thérapeutique dans laquelle le dit principe actif est un corticoïde,
- une méthode de traitement thérapeutique des affections ou traitements vasculaires, veineux ou artériels, dans laquelle le principe actif dans la formulation solide ou semi-solide est introduit dans, ou autour, dé la paroi vasculaire par injection intraiuminale.

Les aspects pharmaceutiques et médicaux de l'invention se rejoignent dans la recherche d'un système fin et miniaturisé pouvant être facilement positionné et activé dans toutes les zones de l'organisme à partir de cathéters d'angiopiastie percutanée translusminale, d'endoscopes ou de tout autre dispositif invasif suffisamment fin et long pour accéder à la zone de dépôt. La forme (fine et longue) de la formulation dans le dispositif d'administration facilite son dépôt local. Cette caractéristique du système sous son aspect pharmaceutique et médical permettra son utilisation générale.

Si on entend par insertion, une forme déposée en surface et par implantation, une injection dans un tissu, le traitement ciblé, voire prolongé, pourra être inséré à l'intérieur d'une cavité naturelle de l'organisme si celle-ci est susceptible de faire office de réservoir naturel, c'est-à-dire si la forme du dépôt de médicament lui permet de séjourner dans la cavité corporelle au moins sur la durée de son relargage. Cette forme pourra être soit la forme allongée étudiée pour faciliter son dépôt avec le dispositif, soit son évolution une fois déposée.

La forme du dispositif et de la formulation n'est donc pas a priori adaptée à la zone d'insertion comme peuvent l'être Ocusert, l'anneau vaginal. ou les stents. La forme de la formulation peut toutefois évoluer après dépôt pour faciliter son maintien local. Après son dépôt, la formulation n'est pas associée à tout ou partie du dispositif de dépôt mais laissée seule au site de dépôt.

Si, pour un besoin et une durée thérapeutique spécifiques, une insertion dans une cavité naturelle de l'organisme n'est pas souhaitable, le traitement ciblé, voire prolongé, pourra également être implanté à l'intérieur d'un tissu cible de l'organisme, pour permettre son dépôt sur la durée de relargage.

Cette implantation pourra être réalisée avec le dispositif associé aux outils classiques, par voie transcutanée, par voie vasculaire ou cavitaire dans une muqueuse ou une paroi de l'organisme ou par voie chirurgicale dans un tissu cible.

L'insertion de la forme retard permettra un traitement local, superficiel ou externe, mais également un ciblage pour effet en profondeur, voire pour effet systémique, par exemple avec un dépôt sur les muqueuses.

De même l'implantation de la forme retard permettra un traitement général mais aussi un traitement ciblé par hyperconcentration local ou par excrétion.

Ainsi, selon les applications thérapeutiques et la zone, l'insertion comme l'implantation pourra être une solution systémique, une solution locale interne ou, enfin, une solution de ciblage externe.

Les formulations solides ou semi-solides immédiates ou retard utilisées dans le procédé de l'invention pourront être n'importe quelles formulations solides ou semi-solides susceptibles de pouvoir être fabriquées ou conditionnées dans la forme et le volume compatibles avec le procédé et le dispositif d'injection.

Ainsi les formulations solides ou semi-solides pourront être préférentiellement des formulations réalisées à partir d'excipients biodégradables comme, par exemple les sels inorganiques (calcium, magnésium, bismuth, zinc); les lipides; les hydrates de carbone (polysaccharides, saccharose, glucose, agarose, dextrine, cyclodextrine et mélange); les protéines (gélatine, collagène modifié, albumine, caséine, dérivés et mélanges) ; les polymères naturels et synthétiques (acide polyisobutyrique, acide polylactique, acide polyglycolique, copolymère polylactide-polyglycolide (PLGA), polyester, polycaprolactone, polyéthylène-glycol, polypropylène-glycol, ®Pluronics. polyanhydrides et leurs mélanges).

Les formulations solides ou semi-solides pourront être réalisées sans excipient ou structurées avec de faibles quantités d'excipient injectable type mannitol, acide hyaluronique, dérivés cellulosiques...

Les formulations semi-solides pourront être réalisées en mélangeant le P.A. avec ou sans excipient, avec de l'eau, un solvant organique, de l'huile ou tout autre liquide injectable susceptible de donner la forme semi-solide.

Les formulations solides ou semi-solides seront soit des formulations immédiates soit des formulations retards.

Les formulations immédiates solides pourront être réalisées comme indiqué dans le brevet SCRAS (Delivery of Solid Drug Compositions WO96/07397). Les formulations semi-solides et solides retards pourront être réalisées selon la formulation et le procédé revendiqué par le brevet SCRAS (Sustained Release of Peptides from Solid and Semi-solid pharmaceutical compositions WO96/07398 dont le contenu est incorporé par référence).

Les formulations solides ou semi-solides seront avantageusement réalisées selon des procédés permettant une forte concentration en principe actif supérieure à 20% voire supérieure à 40%, de préférence supérieure à 50 % et jusqu'à 100% de P.A.

Avant leur dépôt, les formulations solides non dispersées, selon l'invention auront une forme fine et allongée : tige, implant, pellet, bâtonnet ou aiguille, de façon à pouvoir être introduites à l'intérieur du dispositif d'implantation qui pourra lui-même, si nécessaire en fonction de la profondeur de l'injection dans le corps, être à l'intérieur d'un endoscope ou d'un cathéter. Les formulations solides dispersées (poudres, sphères) devront pouvoir être arrangées longitudinalement dans le dispositif.

Les formulations solides dans le dispositif auront, ainsi, préférentiellement un diamètre maximum de 3 mm et avantageusement un diamètre inférieur à 2.5 mm voire un diamètre inférieur à 2 mm, de préférence inférieur à 1 mm. En fonction de la dose totale et surtout pour les formes immédiates ou les formes de courte durée ou de dose faible (inférieure à 0,1mg/jour), le diamètre des formes solides pourra être encore plus petit et jusqu'à 0,1 mm.

Les diamètres les plus petits pourront, dans certains cas présenter un avantage technique pour faciliter l'implantation locale profonde: toutefois, avec les cathéters et les endoscopes, un diamètre plus important n'aura pas les mêmes inconvénients (notamment en terme de confort du malade) que dans le cas des injections superficielles type trocarts (Zoladex, marque déposée par la société Zeneca) ou mini-trocarts (Auto-injecteur, Retro-injecteur : Needle-less Parenteral Introduction DeviceWO96/08289 dont le contenu est incorporé par référence) soit parce que l'utilisation de dispositifs médicaux nécessite, par ailleurs, une anesthésie locale ou générale, soit aussi parce que la zone profonde d'implantation est moins sensible que la peau.

Les formes solides pourront avoir une longueur de quelques centimètres, en général, inférieure à 3 cm et préférentiellement inférieure à 2 cm et adaptée à l'espace dans la zone de dépôt.

Les formes solides seront préférentiellement cylindriques et obtenues par des techniques d'extrusion.

Les formes semi-solides, selon l'invention, auront une viscosité suffisamment élevée pour contenir une forte concentration en PA (préférentiellement supérieure à 20%) et rester homogènes tout en permettant les injections profondes à travers l'aiguille du dispositif de l'invention.

Les formes semi-solides pourront être des gels, des huiles, des pâtes ou toute autre dispersion semi-solide d'un PA dans un véhicule liquide.

Les formes semi-solides auront un volume total faible en général inférieur à 300 µl et préférentiellement inférieur à 100 ul voire inférieur à 50 µl.

Le procédé et les dispositifs selon l'invention utiliseront préférentiellement des excipients injectables biodégradables ou normalement éliminés ou solubilisés dans les fluides corporels.

Toutefois, le procédé pourra utiliser des dispositifs ou formulations à base de biomatériaux biocompatibles non-biodégradables lorsque le site et les outils de dépôt permettront facilement le retrait du dit dispositif ou de la dite formulation après son fonctionnement c'est-à-dire plutôt pour les inserts que pour les implants. Le dispositif ou la formulation devra avoir une forme fine et allongée, comme les autres formes solides compatibles avec l'administration locale profonde. Comme par exemple, les implants silicone Norplant, les systèmes réservoir PHEMA de la société Hydromed, voire les pompes osmotiques Duros de la société Alza.

Les dispositrfs selon l'invention, correspondent aux formulations solides ou semi-solides associées au dispositif d'insertion ou implantation profonde localisée.

Le dispositif selon l'invention, pour l'implantation ou l'insertion d'un principe actif dans une formulation solide ou semi-solide en un site de dépôt précis de l'organisme, est caractérisé en ce qu'il comprend une partie placée à l'intérieur du corps du patient avec des moyens de conditionnement de la forme solide ou semi-solide, des moyens de positionnement jusqu'au site de dépôt, des moyens d'injection ou d'insertion à ce site de dépôt et des moyens de retrait après injection ou insertion, et une partie laissée à l'extérieur avec des moyens d'activation des fonctions du dispositif.

Suivant d'autres particularités :
- les moyens de conditionnement des formes solides ou semi-solides sont aussi les moyens de positionnement et d'injection,
- ledit dispositif comprend un piston à l'intérieur d'un guide pouvant être actionné dans un trocart ou un cathéter,
- le moyen de conditionnement, positionnement, injection est une aiguille,
- la dite aiguille une fois actionnée peut être orientée par rapport au dispositif par préformage ou précontrainte élastique ou par des moyens. mécaniques,
- les moyens externes d'activation du dispositif permettent, de façon séquentielle, l'injection de l'aiguille, l'avancée du piston jusqu'au biseau de l'aiguille pour déposer la forme solide ou semi-solide, le retrait de l'aiguille autour du piston, le retrait combiné de l'aiguille et du piston,
- les actions séquentielles du dispositif à partir. des moyens externes sont contrôlées à distance et en ordre à l'aide de deux butées amovibles dont la première est disposée sur un poussoir coaxial au piston, et la second est une pièce tubulaire intercalée entre le guide et le poussoir,

Les dispositifs pourront être utilisés directement ou associés aux instruments médicaux de thérapie locale (endoscope, fibroscope, tube, cathéter, clou, aérateur, canule, perforateur, trocart...).

Les dispositifs seront introduits au niveau local et permettront l'insertion ou l'implantation des formes semi-solides ou solides Ils seront retirés immédiatement après ce dépôt.

Comme les formulations, les dispositifs utilisés, selon le procédé de l'invention d'administration locale profonde de formulations sotides ou semi-solides, seront polyvalents et de faible volume avec une forme adaptée fine et allongée.

Les dispositifs auront donc, préférentiellement un diamètre maximum de 3 mm et avantageusement un diamètre inférieur à 2,5 mm voire inférieur à 2 mm. En fonction de la formulation, le diamètre du dispositif pourra être encore plus petit et jusqu'à 0,3 mm.

Dans un fibroscope ou un endoscope comportant, par exemple 4 canaux (vidéo, instruments, introduction et retrait de fluide, fibres optiques lumineuses), le dispositif d'insertion ou d'implantation pourra, comme un outil classique (style pince à biopsie) occuper le canal des instruments, ce qui libère le canal d'introduction de fluides voire permet son élimination. Dans ce cas, les dispositifs pourront avoir un diamètre inférieur à 2 mm et, par exemple, de 1,7 mm comme certains instruments.

Dans un cathéter, le dispositif d'insertion ou d'implantation pourra, comme le dispositif d'insertion des stents, occuper le canal et être activé depuis l'extérieur in situ. Dans ce cas, le dispositif pourra avoir un diamètre inférieur à 2,5 mm et, par exemple, de 2 mm comme certains stents.

Dans un trocart, le dispositif d'insertion ou d'implantation pourra, comme le dispositif de perforation, occuper la lumière du trocart. Le dispositif pourra avoir un diamètre inférieur à 3 mm et, par exemple, de 2,5 mm comme certains perforateurs.

D'autres caractéristiques et avantages de l'invention apparaîtront au cours de la description qui va suivre, faite en référence aux dessins annexés qui en illustrent plusieurs modes de réalisation à titre d'exemples non limitatifs.

La figure 1 est une vue en élévation longitudinale d'un premier mode de réalisation du dispositif d'administration de formulations solides conforme à l'invention dans le cas du dépôt de la formulation à l'intérieur d'une cavité naturelle de l'organisme, utilisée comme réservoir de relargage de la formulation.

Les figures 2, 3 et 4 illustrent une séquence de mise en oeuvre du dispositif de la figure 1 pour administrer localement dans l'organisme une formulation solide.

La figure 5 est une vue mi-élévation mi-coupe longitudinale d'un second mode de réalisation du dispositif d'administration de formulations conforme à l'invention, représenté partiellement introduit dans l'organisme d'un patient prêt à l'administration de la formulation solide.

La figure 6 est une vue en coupe transversale suivant 6/6 de la figure 5.

La figure 7 est une vue analogue à la figure 5 montrant le dispositif après poussée de la forme solide à l'extérieur d'un guide du dispositif, prête à être déposée dans l'organisme d'un patient.

La figure 8 est une vue en coupe transversale suivant 8/8 de la figure 7.

La figure 9 est une vue en élévation analogue aux figures 5 et 7. montrant le dispositif après retrait partiel de l'aiguille, la forme solide restant en place dans l'organisme.

La figure 10 est une vue analogue à la figure 9 montrant l'aiguille et le piston intérieur à celui-ci complètement rentrés.

Les figures 11 à 16 sont des vues similaires respectivement aux figures 5 à 10 mais dans lesquelles le dispositif est utilisé pour administrer une forme semi-solide.

Les figures 17, 18 et 19 représentent le relargage in vitro d'inserts de dexaméthasone à une concentration respective de 10, 15 et 20%.

Les figures 20, 21 et 22 représentent les résultats des études pharmaco cinétiques sur le rat d'inserts de dexaméthasones à une concentration respective de 10, 15 et 20% injectés en sous cutané (A) et en intrapéritonéale (B).

Les figures 23 et 24 représentent des résultats des études pharmaco cinétique de la forme solide 12,8 mg d'acétate de lanréotide respectivement sur le chien injectée en intra-musculaire et sur le volontaire sain injectée en sous-cutané (A) et en intra-musculaire (B).

La figure 25 représente l'étude pharmaco cinétique sur le volontaire sain de la forme semi-solide de 40 mg de lanréotide injectée en intra-musculaire.
- la figure 26 représente le profil de relargage in *vitro* d'une formulation matricielle acétate de Triptoréline /PLGA (75:25) à 20 % de principe actif ;
- la figure 27 représente le profil de relargage in *vitro* d'une formulation selon l'invention acétate de Triptoréline/PLGA (75:25) à 52 % de principe actif ;
- la figure 28 représente le profil de relargage *in vitro* d'une formulation pamoate de Triptoréline (principe actif) et PLGA (50:50) à 40 % de principe actif ;
- la figure 29 représente le profil de relargage *in vitro* d'une formulation pamoate de Triptoréline (principe actif) et PLGA (50:50) à 52 % de principe actif ;
- la figure 30 représente des photographies de formulations acétate de Triptoréline PLGA (75:25) à 20 % de principe actif placées dans un milieu physiologique *in vitro* après une heure, 1j, 2j, 3j, 7j et 10 j ;
- la figure 31 représente des photographies de formulations acétate de Triptoréline PLGA (75:25) à 52 % de principe actif placées dans un milieu physiologique *in vitro* après une heure, 1j, 2j, 3j, 7j et 10 j ;
- la figure 32 représente les profils de relargage *in vitro* de trois formes selon l'invention à 52%, 70 % et 80 % de principe actif (acétate de Triptoréline) à la dose de 9 mg ;
- la figure 33 représente les profils de relargage *in vitro* de deux formes selon l'invention à 52 % de principe actif (acétate de Triptoréline) aux doses de 9 mg et 6 mg;
- la figure 34 représente l'évolution au cours du temps des taux de principe actif restant dans des implants injectés chez le rat pour des formulations à 52 %, 70 % et 80 % de principe actif (acétate de Triptoréline);
- la figure 35 représente l'évolution au cours du temps de la quantité résiduelle absolue de principe actif restant dans des implants injectés chez le rat pour des formulations à 52 %, 70 % et 80 % de principe actif (acétate de Triptoréline) ;
- la figure 36 représente les cinétiques chez le chien des concentrations plasmatiques pour une formulation acétate de Triptoréline /PLGA (75:25) à 20 % de principe actif et à la dose de 3 mg et le suivi de l'effet pharmaceutique par le taux de testostérone.
- la figure 37 représente les cinétiques chez le chien des concentrations plasmatiques pour une formulation acétate de Triptoréline /PLGA (75:25) à 52 % de principe actif et à la dose de 6 mg, et le suivi de l'effet pharmaceutique par le taux de testostérone.
- La figure 38 représente les profils de relargage *in vivo* chez le chien d'une formulation acétate de Triptoréline/PLGA (75:25) à 70 % de principe actif et à un dosage de 9 mg (A) et le suivi de l'effet pharmaceutique par le taux de testostérone (B).
- la figure 39 représente les profils de relargage in *vivo* chez le chien de formulation acétate de Triptoréline/PLGA à 52 % de principe actif et à une dose de 6 mg et à 70 % de principe actif et à une dose de 9 mg.

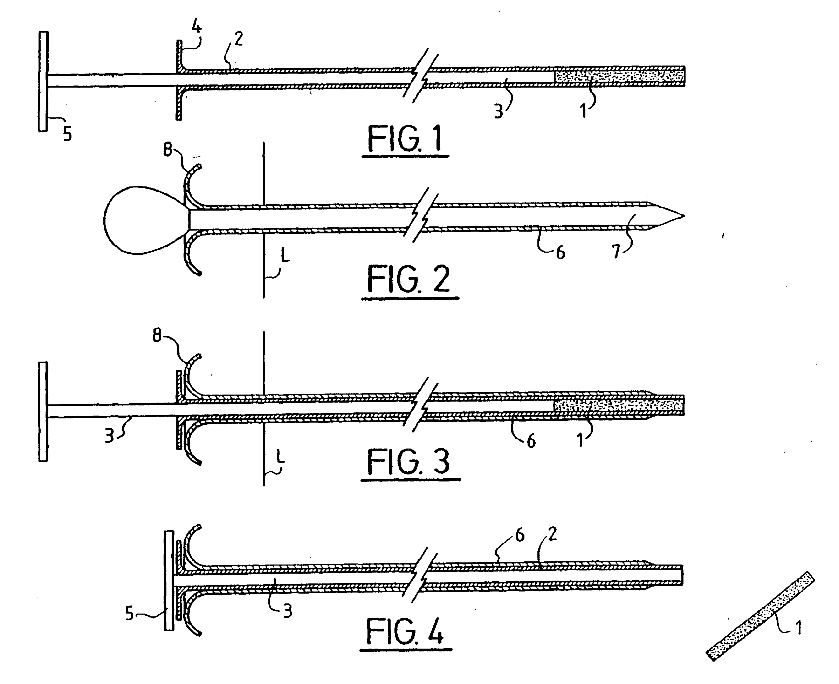

Le dispositif d'administration d'une forme solide 1 représenté à la figure 1 comporte un guide tubulaire 2 contenant un piston 3 pouvant pousser à l'extérieur du guide 2 la forme solide 1 contenue à l'extrémité de ce dernier. Le guide 2 et le piston 3 sont pourvus, à leurs extrémités opposées, de collerettes respectives 4, 5 de manipulation manuelle.

La figure 2 illustre un exemple possible de système invasif dans l'organisme d'un patient pour la mise en oeuvre du dispositif d'administration de la forme solide 1 de la figure 1. Le système invasif est dans l'exemple de la figure 2 un trocart 6 contenant un mandrin perforateur 7, si l'accès à la cavité naturelle de l'organisme utilisé comme réservoir de relargage de la formulation solide 1, nécessite une perforation de tissus internes. Sur la figure 2 le système invasif est représenté partiellement introduit en interne dans l'organisme dans sa partie située à droite du plan L, tandis que sa partie située à gauche reste en externe.

Si l'accès à la cavité naturelle de l'organisme ne nécessite pas une perforation de tissus internes, le système invasif peut être un endoscope, un fibroscope ou un cathéter (non représentés). Le système invasif utilisé est introduit dans la cavité corporelle (sinus de la face, oesophage, trachée, vaisseau...), grâce au mandrin perforateur 7 dans le cas d'un système tel que celui de la figure 2. Puis le mandrin 7 est retiré du trocart 6 (ou de l'endoscope, du cathéter,...) et le dispositif d'administration de la figure 1 est introduit à l'intérieur du trocart 6 (figure 3), jusqu'à ce que la collerette 4 du guide 2 vienne en butée contre l'extrémité annulaire recourbée 8 du trocart 6.

Ensuite il suffit de pousser le piston 3 pour éjecter la forme solide 1 à l'extérieur du guide 2, car aucune résistance tissulaire ne s'oppose à son mouvement (figure 4).

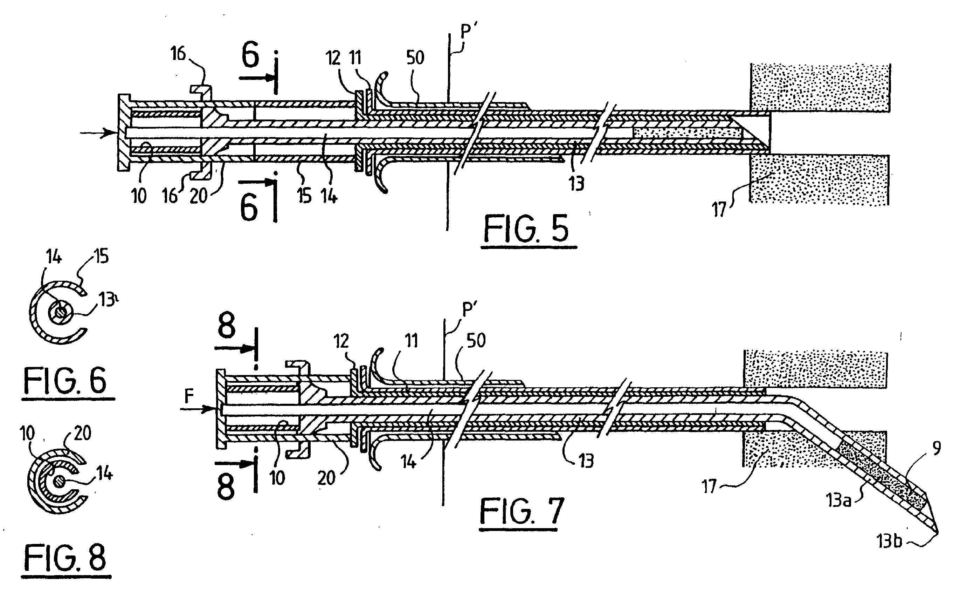

Dans le second mode de réalisation du dispositif d'administration. d'une forme solide 9, illustré aux figures 5 à 10, ce dispositif est prévu pour le cas d'une injection dudit dispositif à l'intérieur d'un tissu, d'une paroi ou d'une muqueuse à partir d'un système invasif interne déjà engagé dans une cavité comme représenté aux dessins, mais aussi à partir d'un système invasif engagé dans un tissu interne.

Le système invasif comprend une pièce tubulaire 50 engagée partiellement dans le tissu à travers la surface P' de celui-ci, et un guide tubulaire 11 qui peut être un fibroscope ou un endoscope, dans lequel peut être monté un cathéter 12. Ce dernier constitue un guide du dispositif d'administration formé par une aiguille 13 et un piston 14 d'extraction de la forme solide 9 dans le tissu 17.

Le dispositif comporte deux butées amovibles (10, 15) dont la première 10 est une douille disposée dans un poussoir 20 coaxial au piston 14, cette butée 10 et le poussoir étant tronqués longitudinalement (figure 8); la seconde est une pièce tubulaire 15, également tronquée (figure 6), interposée entre le cathéter 12 et le poussoir 20.

L'injection du dispositif d'administration 13, 14, 9, peut être obtenue en déplaçant le guide en arrière, mais est réalisée de préférence comme illustré aux figures 7 à 10, de la manière suivante. On retire la butée 15; on déplace l'aiguille 13 à l'aide du poussoir 20 contenant la butée 10. (figure 7). Si nécessaire et comme illustré à la figure 7, notamment dans le cas des vaisseaux, l'aiguille 13 peut présenter à son extrémité une forme courbée 13a obtenue par libération d'une précontrainte élastique de l'aiguille 13 dans le guide. Une fois libérée de la contrainte du guide, l'extrémité courbée 13a facilite l'injection oblique de la forme solide 9 dans la paroi ou la muqueuse 17. Cet angle entre l'aiguille et le guide pourra être obtenu ou réglé, par tout autre mécanisme habituellement utilisé par ces dispositifs.

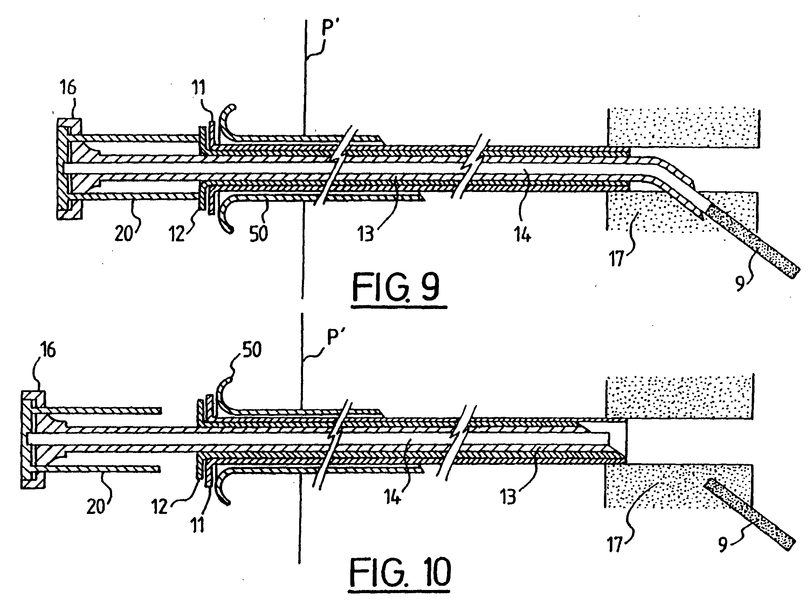

Une fois la forme solide 9 et l'extrémité courbée 13 a injectées, on enlève la butée 10 du poussoir 20, et on rétracte l'aiguille 13 par traction sur les ergots 16 sans déplacement du piston 14, afin de déposer la forme solide 9 dans le tissu 17 (figure 9). Lorsque le biseau 13b de l'aiguille 13 parvient à l'extrémité du piston 14, ce dernier est retiré avec l'aiguille 13, laissant la forme solide 9 en place, cette manoeuvre étant obtenue par traction sur le poussoir 20 et les ergots 16 (figure 10).

Le dispositif des figures 5 à 10 peut également permettre l'administration d'un semi-solide.

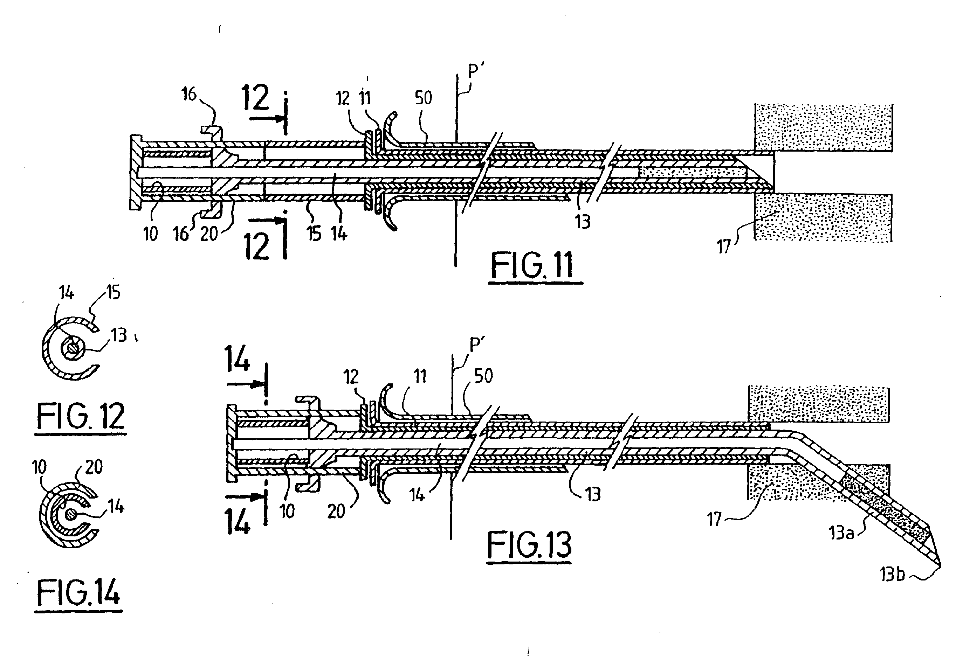

Le dispositif d'administration illustré sur les figures 11 à 16 est similaire à celui des figures 5 à 10 et n'en différe par le fait que le piston 14 agit sur une forme non solide 18, en s'apparentant à une micro-seringue jusqu'à la pointe du dispositif d'injection.

Ici également, le système invasif 9, 11, 12 peut être engagé dans un tissu interne 17.

Le procédé d'administration consiste ici à injecter, en le poussant à l'extérieur du guide 9, 11, 12, le dispositif d'administration constitué de l'aiguille 13, du piston 14 et de la forme semi-solide 18. L'aiguille 13 peut éventuellement être courbée comme dans la réalisation des figures 5 à 10. Le piston 14 est déplacé dans l'aiguille 13 pour injecter le semi-solide 18 (figure 14) de la même manière que dans le mode de réalisation précédent.

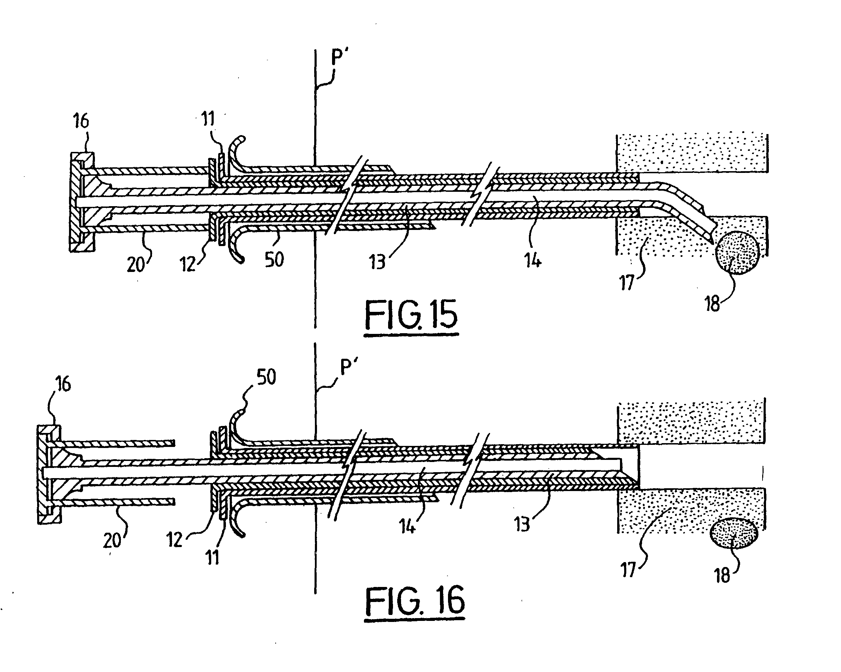

Le piston 14 et l'aiguille 13 sont enfin retirés ensemble par réintroduction dans le guide 11, 12, par traction sur les ergots 16 et sur le poussoir 20 (figures 15 et 16), la forme semi-solide 18 laissée en place dans le tissu 17 pouvant alors prendre une forme sphérique ou ellipsoïde.

Les schémas des figures 1 à 16 vont permettre d'illustrer les procédés d'administration pour différents traitements spécifiques décrits plus loin. Ces différents traitements spécifiques selon le procédé de l'invention d'administration locale solide ou semi-solide impliquent la mise en oeuvre du procédé pour pouvoir être appliqués et offrent, ainsi, certaines solutions thérapeutiques nouvelles qui font partie de l'invention. Ces différents exemples illustrent le champ d'application possible de l'invention, mais ne constituent pas une liste exhaustive d'applications du procédé et ne sont donc pas limitatifs.

Au nombre des traitements possibles, selon le procédé de l'invention, on peut citer les traitements anesthésiques, antalgiques, anti-inflammatoires, cancérologiques, cardiologiques, endocrinologiques, rhumatologiques etc... ainsi que les traitements associés. Au nombre des techniques endoscopiques ou radiologiques susceptibles de permettre ce procédé de traitement local, on peut citer l'urologie, la gynécologie, arthroscopie, l'ORL, la bronchoscopie, la gastrologie, la chirurgie à invasion minimale ou encore le cardio-vasculaire.

Ces procédés sont nouveaux parce qu'ils utilisent une formulation galénique, retard ou non, solide ou semi-solide de faible volume (micro-litres). Cette formulation diffère des traitements locaux existants qui utilisent des formes solides spécifiques ou des formes liquides ou suspension, en grand volume.

Selon ce procédé et avec ces formulations non liquides, la formulation n'est pas, dans sa composition ou dans sa forme, étudiée pour une vectorisation précise. Au contraire, la formulation est étudiée pour un outil ou dispositif adapté à l'administration locale interne et qui permet à distance l'injection ou l'insertion in-situ.

Le procédé pourra utiliser, sous cette forme galénique et avec ces outils, des PA classiques et notamment ceux ayant déjà démontré leur intérêt en administration locale, ou dont l'intérêt local peut être déduit du mode d'action du PA, même si son utilisation sous cette forme n'existe pas encore, notamment parce qu'elle ne pouvait être facilement mise en oeuvre sans la contribution de l'invention. Les différents exemples qui suivent illustrent les possibilités de ce procédé.

Le procédé, les formulations et dispositifs permettent l'administration dans des cavités de l'organisme et en intra-tissulaire. Quelque soit la cavité ou le tissu, l'avantage est de pouvoir amener la formulation au site de dépôt en évitant ou en diminuant les lésions tissulaires.

Ces cavités naturelles pourront être utilisées comme des réservoirs de produit thérapeutique, notamment si leur anatomie permet l"'emprisonnement" de la formulation. Le procédé permet, par exemple, l'administration dans les cavités naturelles de la face et dans ses tissus. Avec certains PA, on retrouve avec ce traitement l'ensemble des objectifs énoncés précédemment (meilleure efficacité locale, diminution de dose, augmentation de la durée d'action, amélioration du confort et de la compliance, diminution des effets secondaires).

Les inserts ou implants intra ou péri-sinusiens pourront véhiculer le PA dans le mucus grâce aux flux ciliaires des muqueuses ou permettre sa diffusion locale systémique par contact. On pourra également envisager une action générale par diffusion progressive vers les voies disgestives pour des médicaments nécessitant des prises quotidiennes à faible dose.

Les corticoïdes locaux sont un bon exemple de produit d'action locale présentant des inconvénients par voie générale. Toutefois, les traitements locaux existants (gouttes, spray...) se heurtent aux dispositions anatomiques pour atteindre une zone cible précise tel que le méat moyen (gouttière unicellulaire). De plus, avec ces traitements existants, la nécessaire persistance du PA en local implique de fréquentes applications.

Ce procédé thérapeutique, selon l'invention, permet d'atteindre cette zone clé de la pathologie naso-sinusienne. En plus des sinus maxillaires, selon les besoins, on-pourra traiter localement les celiules ethmoïdales, les sinus sphénoïdaux et frontaux, la caisse du tympan. La forme retard solide ou semi-solide implantée ou insérée sera en contact avec cette muqueuse qui sécrète et se recouvre d'un mucus circulant du méat vers les fosses nasales, et évacuée vers le cavum en passant au contact du bourrelet tubaire et de la trompe d'Eustache.

Le procédé permettra, par exemple, d'augmenter et de maintenir la concentration de produit thérapeutique dans la gouttière unicellulaire siège des pathologies en particulier inflammatoires. Si la forme non-liquide retard est déposée à l'intérieur des sinus, on utilisera un dispositif selon le schéma de la figure 1 qui pourra être positionné grâce aux outils classiques de drainage de l'oto-rhino-laryngologie (ORL; trocarts, tubes). On pourra également injecter la formulation dans la muqueuse des fosses nasales, dans les cornets ou dans le bourrelet tubaire grâce aux dispositif présentés dans les figures 5 à 16. Selon la. zone de dépôt et la formulation, l'action sera donc préférentiellement externe, intra-tissulaire ou systémique.

En ORL, on pourra ainsi traiter, par exemple, par corticothérapie, la polypose nasosinusienne, les rhinites allergiques ou non, certaines otites ou sinusites non-infectieuses etc. En dehors des traitements anti-inflammatoires, on pourra pratiquer des traitements antibiotiques, anti-allergiques, immunostimulants, etc... On pourra également combiner les traitements. Ces traitements auront une visée locale.

On peut, par exemple, fabriquer des tiges de Dexaméthasone phosphate matricée à hauteur de 15% dans du PLGA 50-50 selon les étapes suivantes pesée des matières premières, mélange des deux poudres, extrusion, dosage, conditionnement et stérilisation. L'implant obtenu pourra avoir un diamètre moyen de 2,4mm pour une longueur de 12,5mm. Il pourra être introduit à l'inténeur des sinus maxillaires à l'aide du dispositif schématisé par la figure 1. II pourra egalement être implanté dans la muqueuse du cornet à l'aide du dispositif schématisé dans les figures 5 à 10.

Cette formulation solide est une formulation retard 1 mois qui contient 7,5mg de Dexaméthasone et qui relargue en moyenne 0,5mg/jour avec un implant dans chaque sinus. Pour un traitement chronique, on pourrait imaginer l'utilisation intra-sinusienne d'une forme polymérique (PLGA 75-25) de trois mois de durée d'action, voire une forme réservoir (type Hydromed) de 1 and de durée d'action.

Ces préparations retard pourront servir en ambulatoire aux patients souffrant, par exemple, d'obstruction nasale chronique. Le geste médical pour l'administration intrasinusienne s'apparentera aux gestes ORL courants, pouvant être pratiqués au cabinet du médecin : ponction au trocart avec ou sans anesthésie. La voie d'abord pourra être préparé ou non (méatotomie, clous, drains ou autres).

L'injection profonde localisée dans le cornet ou les muqueuses des fosses nasales sera également facile grâce au dispositif associé ou non aux outils habituels d'exploration endoscopique. Dans les fosses nasales, l'administration locale est peu profonde. Selon les cavités corporelles ou le site de chirurgie endoscopique, la distance entre la zone externe et le dépôt interne pourra être encore plus courte ou beaucoup plus grande.

On utilise déjà les corticoïdes retard en rhumatologie. On peut, par exemple, selon le procédé, imaginer une injection locale intra ou péri-articulaire avec dépôt sous faible volume de forme retard (corticoïdes, anti-infiammatoires) au site de l'inflammation (tendinite, bursite, arthrite non infectieuses, arthrose...).

On peut aussi, selon le procédé, imaginer un traitement oculaire par injection-dépôt dans la muqueuse sous la paupière. Le faible volume des formes solides ou semi-solides rendront insensible ce dépôt et l'injection favorisera à la fois l'effet retard et le maintien local du traitement de manière plus efficace que le dépôt dans le cul de sac conjonctival qui est abondamment drainé. Cet abord est surtout avantageux pour un traitement chronique comme, par exemple, le glaucome avec la pilocarpine.

Ici, l'injection est pratiquement superficielle et ne nécessite pas d'outils en dehors du dispositif d'administration pour les semi-solides ou solides en micro-volume.

De même, on peut selon le procédé de l'invention traiter certaines tumeurs superficielles ou problèmes cutanés par dépôt local, intra ou hypodermique.

Par exemple, la dermopeptine (BIM 23014C) pourra être utilisée sous une forme retard semi-solide à 20% dans l'eau et sous un volume de 20 microlitres soit une dose totale de 4mg de Somatuline. La formation pourra être injectée au niveau des chéloïdes ou des mélanomes, créant ainsi une concentration locale élevée et prolongée à partir d'une zone de gradient de diffusion au site d'injection.

Dans le cas de certaines tumeurs solides, le traitement pourra être associé à un cytotoxique (Type 5FU ou cisplatine) dont la diffusion sera régulée par la même forme locale et dont la concentration locale sera ainsi très élevée avec une dose totale très basse.

Les mêmes formulations pourront également être utilisées dans les applications beaucoup plus profondes et alors associées à des outils type cathéter actif SMA (Shape memory Alloy) ou fibroscope, et à des spécialités comme la radiologie interventionnelle ou la chirurgie endoscopique ou robotique.

On pourra, par exemple, implanter en intra-cérébral, une forme retard BIM23014 C plus cytotoxique grâce à un accès dans le crâne.

Les formes solides ou semi-solides selon l'invention, présentent l'avantage, par rapport au traitement local type Gliadel, de pouvoir être appliquées sans trépanation au niveau superficiel, mais également en profondeur grâce à la neurochirurgie stéréotaxique, endoscopique et robotique.

Les tumeurs solides traitées, par exemple, avec les formes collagènes, de Matrix pourront être traitées de même avec ces microdosages. Quelle que soit la forme solide ou semi-solide, l'avantage du volume permet la vectorisation dans tous les sites et évite le risque de propagation entraînée par l'injection d'un volume liquide de quelques millilitres.

Toujours avec une forme solide ou semi-solide localisée plus profondément dans l'organisme, après angioplastie percutanée transluminale, on peut traiter la resténose en local intra-vasculaire. Par rapport au traitement local associé au stent, l'avantage d'un traitement selon le procédé est de ne pas être confronté à la limite de dose de l'espace vasculaire et de la surface du dispositif, et de ne pas entrer en contact direct avec la paroi vasculaire lésée, tout en permettant une forte concentration locale dans toutes les couches du vaisseau et autour, et un effet systémique si nécessaire.

On pourra, par exemple, injecter selon le schéma des figures 5 à 16, l'Angiopeptine seule ou associée à l'Héparine. On pourra bien sûr injecter tout autre PA seul ou associé susceptible d'éviter les risques de resténose et, de favoriser le résultat.

Par rapport à cette thérapie périvasculaire, on peut également mentionner l'utilisation possible des formes semi-solides en intra-vasculaire avec le même objectif que la chimio-embolisation par suspension, colle ou gel.. L'avantage est, ici, d'utiliser une forme retard dont le volume (donc la zone de dépôt) est préfixé; ce qui permet une meilleure localisation de l'occlusion dans le vaisseau.

Le procédé et les dispositifs, selon l'invention, associés au fibroscope ou à toute autre solution d'imagerie directe ou indirecte, permettent l'administration dans les parois organiques.

Par exemple puisque l'on intervient au niveau de la vessie par voie urétrale, on peut imaginer l'implantation d'un traitement (prophylaxie, antibiotique...) dans l'épaisseur de l'urètre.

On peut accéder à la trachée artère et aux bronches (stents). On pourra donc envisager, selon le procédé, un traitement pulmonaire soit par dépôt d'une forme solide ou semi-solide dans le poumon soit par implantation dans la muqueuse, des bronches ou de la trachée, selon les besoins de tolérance locale en intra-pulmonaire, la forme solide pourra être dispersée (poudre ou sphère).

Par exemple, pour remplacer le traitement préventif par glucocorticostéroïdes inhalés dans l'asthme léger ou modéré de diagnostique récent. On pourra administrer dans le poumon, par les bronches, ou dans la paroi qui les recouvre ou dans celles de la trachée, une forme retard de 0,4mg de Budésonide quotidienne qui sera sécrétée dans le flux si la forme est implantée, et qui sera véhiculée par l'humidité jusqu'au fond des alvéoles pulmonaires. Ce traitement préventif à dose basse, sans effet secondaire, ne pose plus ainsi de problème d'observance notamment chez l'enfant. Une telle forme pourra avoir une durée de 1 à 3 mois, voire plus si nécessaire.

Dans le tractus digestif, on dispose également de dispositifs permettant l'administration locale de traitement selon l'invention.

Dans l'oesophage et dans l'estomac le traitement des varices pourra être envisagé à partir d'une forme locale et injectée dans la paroi. De même, les tumeurs à ce niveau qui sont bien individualisées et sont actuellement traitées, par exemple par PDT (photochimiothérapie), nécessitent après injection du produit photosensible, l'illumination par introduction contrôlée d'un diffuseur de lumière au niveau local. Il est donc également possible d'injecter directement les anticancéreux à ce niveau sous forme solide ou semi-solide avec les dispositifs de l'invention. On peut, alors, cibler encore davantage la zone à traiter et éviter de léser inutilement les tissus périphériques.

Le procédé d'administration locale de formes solides ou semi-solides, implique la présence prolongée d'un dépôt local de PA. On peut, si nécessaire, envisager d'ajouter à la formulation des produits favorisant la tolérance locale au site de dépôt. On pourra, par exemple, ajouter de très faible pourcentage de Dexaméthasone, d'indométhacine, d'Héparine ou tout autre PA susceptible d'éviter un effet local indésirable.

Les muqueuses ou les parois sont plus perméables que la peau et il existe des systèmes de patchs ou de bioadhésifs qui s'appliquent sur les muqueuses (notamment buccales ou nasales) et qui permettent un passage systémique du PA L'inconvénient est parfois la non persistance de la formulation au contact de la muqueuse. La présence prolongée de l'administration selon le procédé au niveau local des muqueuses ou des parois internes pourra donc présenter un avantage dans la recherche d'une forme topique à activité systémique. On pourra donc, selon le traitement local, ajouter à la formulation en faible quantité tout excipient susceptible d'être un vecteur de pénétration tissulaire adaptée aux PA (solvants organiques, tensioactifs...). Ainsi, une forme locale profonde pourra avantageusement être le site d'une diffusion systémique par rapport à la muqueuse buccale ou nasale, par exemple, qui ne permettrait pas un dépôt topique prolongé.

Le procédé selon l'invention trouvera également son application durant les interventions de chirurgie à invasion minimale endoscopique (laparoscopique, arthroscopique, etc). Les PA utilisés (anesthésiques locaux, anticoagulants...) peuvent être administrés sous une forme solide ou semi-solide avec ici, encore, l'avantage d'un micro-volume en adéquation avec l'espace réduit d'intervention, et la possibilité d'administration par la voie d'accès instrumentale.

On pourra bien sûr implanter grâce au procédé toute autre forme. retard solide ou semi-solide et notamment des implants PLGA. On peut les utiliser avec d'autres peptides, des protéines recombinantes (interféron), des anticorps polyclonaux ou monoclonaux, des oligonucléotides ou polynucléotides antisens, etc..

Les formulations solides ou implants pouvant être utilisés pour une administration locale du principe actif comme décrit ci-dessus conviennent également, de par leur forme longue et fine et leur faible diamètre, à d'autres types d'administrations classiques par exemple pour traitement systémique par injection cutanée ou intramusculaire.

On a aussi constaté de manière tout à fait inattendue, que lesdites formulations solides ou implants, notamment avec un excipient PLGA, ayant une concentration très importante de principe actif, telle que décrite ci-dessus, qu'il soit soluble ou insoluble, et notamment celles ayant une concentration en principe actif comprise entre 40 et 100 %, et de préférence supérieure à 50 %, permettent d'obtenir *in vivo,* des durées de relargage extrêmement longues, de un mois à trois mois et davantage, et des taux de reiargage très réguliers, voire constants, en étant réalisées sous forme allongée fine, de diamètre ou encombrement transversal inférieur ou égal à 3 mm, par exemple 2,5 ou 2 mm, voire 1 mm ou moins, alors qu'elles se dissolvent très rapidement *in vitro,* et ceci qu'elles soient utilisées pour une action locale ou non.

Classiquement, de tels taux de principe actif étaient prévus pour des formulations à libération instantanée ou rapide.

Les inventeurs ont en outre découvert que, sous une certaine forme, en répartition homogène d'excipient, notamment de PLGA, il était possible d'obtenir une formulation retard selon un mode non matriciel dans lequel le rôle joué par l'excipient était différent ; ce qui conduit à des formulations plus avantageuses dont les caractéristiques sont différentes, ce qui les distinguent nettement des formes matricielles existantes.

Ces formes non matricielles peuvent être qualifiées de formes matricielles de principe actif dans lequel l'excipient est dispersé.

Les formes matricielles mettant en oeuvre le PLGA utilisées à ce jour peuvent être, soit des formes dispersées (microparticules), soit des formes non dispersées (implants).

De manière générale, parmi les formulations retard qui ont été développées, on trouve des formes dites réservoir et des formes matricielles.

Les formes "réservoir" utilisent une barrière ou membrane de diffusion entre le principe actif et le milieu qui va servir à réguler le relargage de ce principe actif. Le médicament peut être à l'intérieur du réservoir sous une forme solide, semi-solide ou liquide. Il peut être en solution ou dispersé dans un excipient. La membrane assure par sa porosité le passage contrôlé du principe actif vers l'extérieur. Parmi les systèmes "réservoir" pour des principes actifs solubles, on peut citer les membranes hydrophiles en poly-méthacrylate d'hydroxyéthyle réticulées (pHEMA, Hydro Med Sciences). Les formes "réservoir" permettent d'obtenir un niveau de reiargage relativement constant d'ordre 0. L'inconvénient principal de ces techniques réservoir est la nécessité de retirer l'implant biocompatible mais non-biodégradable après libération du principe actif.

Les formes matricielles utilisent une matrice ou trame polymère dans laquelle le principe actif est emprisonné pour être libéré par diffusion, par érosion ou par combinaison des deux phénomènes.

Les formes matricielles non-biodégradables, comme par exemple les implants en polymère hydrophobe de type silicone PDMS (Norplant, hormones progestatives), fonctionnent seulement par diffusion. Ce mode de fonctionnement peut entraîner un relargage décroissant d'ordre 1 lorsque la distance de diffusion augmente. L'inconvénient est ici encore la nécessité de retirer l'implant silicone une fois libéré le principe actif.

Les formes matricielles biodégradables ne présentent, en revanche, pas cet inconvénient puisque la matrice polymère est éliminée par l'organisme. De plus, cette élimination ou érosion peut participer à la régulation du relargage du principe actif pour obtenir un relargage constant.

Les formes biodégradables matricielles les plus répandues actuellement utilisent les polymères d'acide lactique ou d'acide glycolique, les copolymères d'acide lactique et d'acide glycolique (PLGA) ou leurs mélanges.

Ainsi, EP 52510 dont le contenu est incorporé par référence décrit une formulation PLGA avec encapsulation de LHRH ou analogue qui peut être une forme dispersée de microcapsules réalisées par coacervation dont la particularité est la répartition du principe actif au centre de la microcapsule avec une couche de PLGA périphérique.

De EP 58481 dont le contenu est incorporé par référence, on connaît des formulations de peptides et de PLGA, dispersées ou non dispersées, tels des implants, dans lesquels le principe actif est uniformément réparti jusqu'à la surface et utilisant un PLGA spécifique de façon à ce que les deux phases de relargage (diffusion et dégradation) se chevauchent sans qu'il y ait d'interruption dans le relargage du principe actif.

De nombreux autres documents portent sur l'utilisation du PLGA dans des formulations retard pour des peptides mais aussi des protéines et des gènes. Le brevet WO 96/40072 dont le contenu est incorporé par référence décrit ainsi une préparation d'hormone de croissance humaine dont la stabilité dans la matrice et dans les solvants organiques utilisés pour la microencapsulation est favorisée et dont le relargage est assuré par la matrice PLGA. Le procédé de contrôle est basé sur la dégradation du polymère et l'ouverture de pores dans la structure qu'elle entraîne.

Tous les travaux effectués à ce jour concordent pour dire que le procédé de contrôle retard avec le PLGA peut entraîner jusqu'à trois phases de libération. Une phase initiale qui libère le principe actif par diffusion, une phase de latence où aucun relargage n'intervient, et une phase de relargage des formes liées corrélée avec la perte de masse du polymère.

Dans toutes les formulations utilisant le PLGA, le contrôle de l'effet retard est obtenu par mélange matriciel du PLGA et du principe actif de façon à permettre à la matrice polymère de jouer son rôle de barrière de relargage du principe actif, voire un rôle dans les interactions physicochimiques entre le principe actif et la matrice polymère.

Dans tous les cas, ce mode de relargage requiert une dispersion du principe actif dans la matrice polymère biodégradable, de façon à isoler les zones de charges en principe actif au milieu extérieur et les retenir au sein de la matrice jusqu'à ce que la biodégradation de celle-ci libère le principe actif qui pourra alors diffuser vers l'extérieur.

Ce type de forme retard matricielle peut être aisément caractérisé en y faisant pénétrer de l'eau qui va hydrater les zones dispersées de principe actif et provoquer un gonflement de la formulation sous l'effet de l'hydratation par les forces osmotiques, du fait de l'impossibilité du principe actif à s'échapper de la structure matricielle.

Ces phases s'entremêlent plus ou moins selon les formulation PLGA, la dégradation du polymère permettant par exemple l'augmentation de la taille des cavités par lesquelles le principe actif peut diffuser.

En dehors du PLGA et des acides polylactiques mentionnés précédemment, il existe assez peu d'excipients retard injectables. On peut citer néanmoins, par exemple, certains polymères, gels et corps gras. Les polyanhydrides sont par exemple des polymères dont le procédé d'érosion de surface donne un profil de relargage distinct de celui du PLGA et plus dépendant de la forme du dépôt que le PLGA qui subit une dégradation globale.

On trouve également certaines formulations retard qui utilisent le collagène ou la gélatine pour obtenir un relargage dans le temps. D'autres formulations utilisent des gels ou des hydrogels. Matrix Pharmaceutical® , par exemple, utilise un gel injectable (AccuSite® , Intradose® ) visqueux.

Ces formulations constituées d'une matrice moins susceptible d'isoler le principe actif du milieu ou plus rapidement éliminée, contiennent en général un faible pourcentage de principe actif.

D'autres excipients injectables comme le mannitol, les polyéthylèneglycols, l'acide hyaluronique sont également utilisés, plus souvent comme additifs pour ajuster le profil retard.

En dehors des techniques matricielles ou réservoir, peu d'autres approches permettent aujourd'hui d'obtenir un relargage suffisamment long, régulier et précis.

On peut cependant citer le cas des implants totalement ou partiellement recouverts d'un revêtement ("coating") servant de barrière à la diffusion du principe actif.

Dans les formulations matricielles dispersées ou non, une certaine quantité de principe actif est retrouvée à la surface de la formulation et n'est pas englobée dans la matrice polymère.

Dans les formes matricielles dispersées, pour une quantité donnée de principe actif, le principe actif de surface représente une quantité relative élevée par rapport au total du principe actif en raison de l'importance de la surface par rapport au volume total.

Pour augmenter la charge ou "core-loading" (C.L.) en principe actif, on est donc contraint d'injecter un grand volume de matrice polymère pour un volume de principe actif donné.

Cette contrainte est encore plus pénalisante pour les formes non dispersées ou implants car le volume de celles-ci pour une quantité de charge élevée, nécessite l'utilisation d'un trocart pour l'injection de la formulation.

On a bien évidemment cherché à concevoir des formulations ayant un C.L. plus élevé, mais l'expérience a démontré l'existence d'un phénomène connu sous le nom de percolation qui se traduit par un relargage rapide de la quasi-totalité du principe actif du fait que dans la matrice polymère, les zones de charge se trouvent en contact les unes aux autres, le polymère (PLGA) n'assurant plus sa fonction de matrice.

Sur le plan visuel, ce phénomène se traduit après hydratation de la formulation par une libération dans un délai très court de principe actif sans gonflement de la formulation, le principe actif étant entraîné à l'extérieur de la formulation par l'eau qui circule dans la matrice polymère.

Dans les formes retard matricielles, le type de PLGA et ses caractéristiques physico-chimique sont clairement précisés et déterminent un domaine de faisabilité. L'influence directe du PLGA sur le relargage par son rôle de barrière matricielle, son rôle dans les relations (hydrophobe, hydrophile, etc...) avec le principe actif et l'influence de sa dégradation impliquent un choix précis de PLGA.

Cette relation entre le PLGA et le relargage apparaît nettement, par exemple, dans la durée d'action d'une formulation matricielle.

Dans une telle formulation, la durée de relargage dépend directement du temps de dégradation du PLGA (deuxième phase ou rebond). Ainsi, on va sélectionner les PLGA en fonction de la durée recherchée. Par exemple, les PLGA 50:50 dépolymérisés en un mois, seront utilisés pour réaliser une formulation un mois alors que nécessairement des formuiations sur trois mois impliqueront des PLGA dont l'hydrolyse est plus tardive, par exemple, des PLGA 75:25.

Dans les formulations non matricielles de l'invention, l'excipient notamment le PLGA, n'influence pas le relargage et il est, par exemple, possible d'obtenir des relargages sur trois mois avec un seul PLGA 50:50 qui disparaît totalement de l'organisme en 60 jours ou encore des formes un mois avec un PLGA 75:25 qui n'aura pas encore commencé son hydrolyse alors que tout le principe actif sera libéré. Ceci est rendu possible par le fait que la proportion de PLGA est toujours inférieure en poids à la proportion de principe actif ; ce qui signifie que la matrice continue n'est plus le PLGA mais le principe actif qui va donc subir pour l'ensemble de la charge, l'influence extérieure et notamment aqueuse. C'est donc le principe actif, notamment par la quantité totale, qui va déterminer la durée d'action.

L'invention a donc aussi notamment pour objet de telles formulations, qu'elles soient à visée systémique ou pour un traitement local, avec un dosage classique ou diminué pour une action locale.

L'invention a plus particulièrement pour objet une formulation retard pour administration parentérale comprenant un mélange homogène d'un principe actif à l'état non dispersé formant une phase continue dont au moins une partie est en contact direct avec la surface d'échange de la formulation et du milieu biologique extérieur, et d'un excipient biocompatible Diodégradable, dans laquelle la quantité de principe actif est d'au moins 50 % en poids par rapport au poids total de la formulation, et ayant un profil de relargage qui est indépendant de la composition de l'excipient du poids moléculaire de l'excipient ou du rapport pondéral principe actif/excipient, le profil de relargage étant essentiellement exclusivement dépendant de la quantité totale de principe actif présent dans la formulation.

A l'inverse des formes matricielles connues autorisant une "charge en principe actif' dans une limite supérieure de 30 % de principe actif afin d'éviter les phénomènes de percolation, les formulations de l'invention contiennent plus de 50 % de principe actif, ce qui représente une diminution en volume du dépôt de l'ordre de 3 à 10 fois par rapport au volume des formes matricielles.

Ainsi, sous forme solide, les formulations de l'invention contiennent avantageusement, avant comme après dépôt, au moins 50 %, de préférence au moins 51 %, avantageusement au moins 60 % et plus avantageusement au moins 70 % et jusqu'à 99,999 % en poids par rapport au poids total de la formulation, l'excipient représentant moins de 50 %, de préférence moins de 49 %, et plus avantageusement moins de 30 % en poids par rapport au poids total de la formulation.

Les excipients sont ceux traditionnellement utilisés dans les formes à libération retard solides, notamment les polymères biodégradables.

On peut citer à titre d'exemple, les polymères de type acide polylactique ou polyglycolique, ou les copolymères de type acide polylactique-polyglycolique, ou des mélanges de ces polymères et/ou. copolymères.

Le choix du polymère biocompatible biodégradable formant l'excipient est indifférent, celui-ci n'influant pas sur la capacité de diffusion du principe actif dans le polymère.

On pourra, par exemple, utiliser un corps gras injectable comme excipient des formulations de l'invention, tel qu'un acide gras polymérisé ou saturé comme le ®Compritol ou encore des excipients comme la polyvinylpyrrolidone (PVP) ou le polyéthylèneglycol (PEG).

La viscosité des polymères peut varier considérablement. Il a été montré que des polymères de basse viscosité peuvent convenir à un mode de relargage dit monophasique de principe actif. Les brevets EP 58481 et 52510 précités mais aussi les brevets EP 21234 et EP 26599 dont les contenus sont incorporés par référence, par exemple, ont mis l'accent sur des polymères de faible viscosité. Ces polymères peuvent convenir à la présente invention (par exemple, viscosité inférieure à 0,5 dl/g dans le chloroforme). La Demanderesse par ailleurs a montré dans une demande de brevet déjà déposée FR 97 04 837 et dans les exemples ci-après que de façon inattendue des polymères de haute viscosité, notamment supérieure à 0,5 voire à 0,6 et pouvant aller jusqu'à 1,2 dl/g peuvent convenir et de façon préférentielle pour obtenir une libération monophasique.

On peut utiliser des DL-PLGA ou des L-PLGA, plus préférentiellement un DL-PLGA réalisé à partir de 70 à 80 % de DL-lactide et de 20 à 30 % de glycolide. Un PLGA synthétisé à partir de 75 % de DL-lactide à 25 % de glycolide convient particulièrement bien mais d'autres copolymères y compris des PLGA 50-50 peuvent également être utilisés. On peut également utiliser des polymères D ou DL-lactide.

Les PLGA peuvent être hydrophiles ou hydrophobes. On peut réaliser les formulations de l'invention avec les polymères hydrophiles.

A titre de polymère biocompatible biodégradable, on préfère toutefois le PLGA, notamment un PLGA hydrophile ayant avantageusement une viscosité dans le chloroforme à 1 g/100 ml supérieure à 0,6 dl/g.

La durée d'action de la formulation retard sera déterminée exclusivement par la quantité totale de principe actif qu'elle contient.

Par principe actif à l'état non dispersé, on entend que les différentes particules de principe actif présentes dans la formulation sont en majorité physiquement en contact les unes avec les autres et jusqu'à la surface de la formulation.

On comprend donc que par phase continue, on entend une distribution telle que toutes ou la plupart des parties internes du principe actif ne sont séparées de la surface que par du principe actif ou un mélange de principe actif et d'une substance ne s'opposant pas à la diffusion ou la dissolution du principe actif.

Avantageusement, le mélange formé par le principe actif et l'excipient est en tous points homogène.

Les formulations retard selon l'invention sont en outre caractérisées par leur différence de durée de relargage *in vitro* et *in vivo.*

Ainsi, les formulations selon l'invention placées dans un milieu aqueux physiologique relarguent le principe actif sur une période inférieure à sept jours alors que la durée d'action *in vivo* est substantiellement supérieure à cette période, avantageusement d'un mois au moins, et de préférence d'au moins trois mois.

Les formulations matricielles comprenant la même quantité de principe actif présentaient à l'inverse un relargage *in vitro* plus long, du même ordre de grandeur que la durée de relargage *in vivo.*

De façon surprenante, malgré un relargage *in vitro* d'une durée limitée, les formulations selon l'invention permettent d'obtenir *in vivo* une durée de relargage largement supérieure sans relation avec la durée de relargage *in vitro.*

En outre, le profil de relargage *in vivo* est clairement différent de celui des formes matricielles en deux phases et sera de pseudo ordre 0, correspondant à une diffusion constante du principe actif au cours du temps.

Ce profil de relargage constitue un autre avantage puisqu'il permet une libération de principe actif d'un niveau constant dans l'organisme.

Les formulations selon l'invention sont injectées directement sous leur forme solide en l'absence de tout excipient liquide ; la proportion élevée de principe actif constitue donc un avantage déterminant, en permettant de réduire le volume de façon importante.

Ainsi, par rapport à une forme matricielle à 20 % de principe actif, les nouvelles formulations selon l'invention à par exemple 70 % de principe actif permettent de réduire le volume d'un facteur de 3,5 ou encore de multiplier la dose par 3,5 pour un volume identique.

Ceci signifie que là où, pour un principe actif donné avec une formulation matricielle non dispersée, un trocart était nécessaire pour injecter un implant d'un diamètre supérieur à 1,8 mm, il suffit d'une aiguille intramusculaire standard pour déposer un microimplant d'une formulation selon l'invention ayant moins de 1 mm de diamètre.

En outre, le mode de relargage de la formulation de l'invention, sans absorption de fluides, ni gonflement initial d'une matrice, constitue un avantage de stabilité pour le principe actif qui est conservé dans un environnement contrôlé. Les formes retard, selon l'invention, sont donc particulièrement avantageuses pour les principes actifs fragiles tels que les protéines recombinantes.

Dans la mesure où il n'existe pas de limitation pour le principe actif tenant compte de la nature du polymère biocompatible biodégradable formant l'excipient, il est possible d'incorporer dans les formulations selon l'invention des principes actifs de haut poids moléculaire qui n'étaient pas capables de diffuser dans les formes matricielles de l'état de la technique, notamment des macromolécules synthétiques ou naturelles, notamment les protéines, ou leurs analogues.

L'invention permet ainsi la libération sur une durée prolongée de molécules fragiles, notamment de peptides et de protéines, ou leurs analogues.

Parmi les substances actives utilisables pour l'invention, on peut notamment citer les protéines, les peptides choisis par exemple dans le groupe constitué de l'acétate de Triptoréline, l'acétate de lanréotide, d'un composé ayant une activité LH-RH tel que la Triptoréline, la goséréline, la leuproréline, la buséréline ou leurs sels, un antagoniste de LH-RH, un antagoniste de GPllb/llla, un composé ayant une activité similaire à un antagoniste de GPllb/llla, l'érythropoiétine (EPO) ou un de ses analogues, les différents interférons α, l'interféron β ou γ, la somatostatine, un dérivé de la somatostatine tel que décrit dans le brevet européen EP 215171 dont le contenu est incorporé par référence, un analogue de la somatostatine tel que décrit dans le brevet américain US 5,552,520 (ce brevet comporte lui-même une liste d'autres brevets décrivant des analogues de la somatostatine qui sont incorporés par référence à la présente demande), l'insuline, une hormone de croissance, un facteur libérateur d'hormone de croissance (GRF), un facteur de croissance épidermique (EGF), une hormone mélanocyte-stimulante (MSH), une hormone libératrice de thyrotropine (TRH) ou un de ses sels ou dérivés, une hormone stimulant la thyroïde (TSH), une hormone lutéinisante (LH), une hormone stimulant les follicules (FSH), une hormone parathyroïdienne (PTH) ou un de ses dérivés, un hydrochlorure de lysozyme, un fragment de peptide à extrémité N-terminale (position 1→34) de l'hormone PTH humaine, la vasopressine ou un de ses dérivés, l'oxytocine, la calcitonine, un dérivé de la calcitonine ayant une activité similaire à celle de la calcitonine, le glucagon, la gastrine, la sécrétine, la pancréozymine, la choiécystokinine, l'angiotensine, le lactogène du placenta humain, la gonadotropine chorionique humaine (HCG), l'enképhaline, le facteur stimulateur de colonies (CSF), un dérivé de l'enképhaline, l'endorphine, la kyotorphine, les interleukines, par exemple l'Interleukine 2, la tuftsine, la thymopoiétine, la thymosthymline, le facteur thymique humoral (THF), le facteur thymique sérique (FTS), un dérivé du facteur thymique sérique (FTS), la thymosine, le facteur thymique X, le facteur de nécrose tumorale (TNF), la motiline, la bombesine ou un de ses. dérivés tels que décrits dans le brevet américain US 5,552,520 (ce brevet comporte lui-même une liste d'autres brevets décrivant des dérivés de la bombesine qui sont incorporés par référence à la présente demande), la prolactine, la neurotensine, la dynorphine, la caeruléine, la substance P, l'urokinase, l'asparaginase, la bradykinine, la kallikréine, le facteur de croissance nerveuse, un facteur de coagulation sanguine, la polymixine B, la colistine, la gramicine, la bacitracine, un peptide stimulant la synthèse protéique, un antagoniste de l'endothéline ou un de ses sels ou dérivés, un polypeptide intestinal vasoactif (VIP), l'hormone adrénocorticotropique (ACTH), un facteur de croissance dérivé des plaquettes (PDGF), une protéine morphogénétique osseuse (BMP), et un polypeptide inhibiteur gastrique (GIP). Toute autre substance active hydrosoluble, ou un de ses sels ou précurseurs, pourra également être utilisée par l'homme du métier s'il le juge utile.

De préférence, on utilisera un produit hydrosoluble, obtenu par salification sous forme de cation, avec par exemple l'acide acétique. On peut cependant utiliser un sel insoluble, comme le pamoate.

Par peptide et/ou protéine, on entend aussi bien le peptide et/ou la protéine eux-mêmes que des fragments de ces peptides ou protéines pharmacologiquement actifs.

La substance active hydrosoluble telle qu'utilisée pour fabriquer les formulations ou implants selon l'invention peut être en particulier l'acétate de Triptoréline, l'acétate de lanréotide, la goséretine, la leuproréline, la buséréline ou leurs sels.

Ces formulations présentent en outre l'avantage de pouvoir être administrées grâce à l'utilisation du dispositif ci-dessus pour le procédé selon l'invention.

Les procédés de fabrication des formulations selon l'invention relèvent de techniques de mélange, de techniques de compression, de techniques d'extrusion à l'état fondu et des techniques de moulage, classiquement utilisées dans le domaine de la fabrication de formes galéniques retard.

L'invention a également pour objet un procédé de préparation d'une formulation retard selon l'invention comportant les étapes consistant à:
- réaliser un mélange homogène du principe actif et de l'excipient, contenant au moins 50 % de principe actif ;
- compacter ledit mélange ; et
- extruder ledit mélange compacté à l'état fondu.

Un procédé alternatif s'appliquant de façon générale aux compositions matricielles et non matricielles, quelle que soit la teneur en principe actif et en excipient, notamment en PLGA, et destinées aussi bien à une application locale que non locale et ne nécessitant ni solvant, ni chauffage du mélange, comprend les étapes consistant à :
- réaliser un mélange homogène du principe actif et de l'excipient ;
- soumettre le mélange homogène à une compression élevée de préférence avec une force supérieure à 1000 kg ;
- broyer les comprimés obtenus ; et
- mettre sous une forme adaptée à l'administration.

Selon le premier procédé, on procède par exemple de la manière suivante :

On pèse le principe actif (PA) et le PLGA aux proportions en poids du mélange (par exemple 70 % PA et 30 % PLGA).

On mélange pour obtenir un mélange homogène, par exemple à l'aide d'un mélangeur ®Turbulat. On charge ensuite le mélange dans une matrice de compression.

On procède à une compactation qui correspond, en fait, à une compression "douce" qui permet de former des briquettes de, par exemple, 13 mm de diamètre sur 5 mm d'épaisseur. Cela est réalisé avantageusement avec une presse à genouillère.

On procède au broyage des briquettes qui peut, par exemple, être réalisé par un tamisage, un cryo-broyage à boules ou avec un broyeur à couteaux.

Cette opération a pour objectif d'améliorer la qualité du flux de mélange de poudre durant l'extrusion nécessaire dans cette situation particulière où la partie fondue représente moins de 50 % du total.

On extrude le mélange à travers une filière de même diamètre que les microimplants souhaités. On récupère l'extrudât après contrôle du diamètre par rayons laser (Keyence) sur une chenillette d'étirage.

Préférentiellement, les microimplants sont calibrés par la buse d'extrusion et non par étirage.

On coupe l'extrudât à la longueur voulue en fonction du contrôle analytique pour obtenir les microimplants qui sont ensuite chargés dans les dispositifs d'injection avant gamma-irradiation (25 kGy).

Selon le second procédé, on procède par exemple comme suit:

A partir d'un mélange de PA et PLGA, on procède non plus à une simple compactation mais à une compression très élevée du mélange à partir des mêmes constituants (excipients et principe actif).

Cette hypercompression pourra être obtenue avec une force minimum d'une tonne.

La conséquence de cette hypercompression réalisée à un diamètre important, par exemple de 13 mm ou supérieur, est la transformation de cet excipient thermoplastique (susceptible de fondre à la température) en une structure semblable à celle obtenue à chaud, c'est-à-dire transparente ou vitreuse, très différente de la précédente obtenue après simple compactation.

Cette opération a lieu à température ambiante, à froid ou même inférieur à 0°C. Durant cette hypercompression, on obtient, à une température inférieure, la transition vitreuse vers l'état plastique de l'excipient au sein du mélange.

Ces hyper-comprimés ensuite broyés comme précédemment pourront être recompactés sous la forme de micro-comprimés équivalents aux microimplants précédents.

Cette technique particulièrement adaptée aux formes retard de PLGA permet sans température, ni solvant, ni véhicules de fabrication, l'obtention de formes galéniques particulièrement intéressantes pour préserver l'intégrité du principe actif, notamment pour les molécules fragiles comme, par exemple, les protéines recombinantes.

Ce procédé est également intéressant pour la fabrication de formes matricielles (ne comprenant pas plus de 50 % de principe actif) que celles-ci soient dispersées ou non dispersées. Pour les formes matricielles la compression du PLGA conduit à une structure matricielle équivalente à celle obtenue en faisant fondre l'excipient à chaud.

Les hyper-comprimés après broyage peuvent être utilisés directement sous une forme dispersée de micro-particules.

La forme dispersée pourra être injectée directement après chargement dans une aiguille d'un dispositif tel que décrit ci-dessus soit être injectée en suspension dans un milieu liquide (comme pour les microsphères, par exemple).

Un des aspects possibles pour la forme solide est celui d'un cylindre allongé.

La formulation telle que définie ci-dessus peut, de préférence, avoir les formes et dimensions définies précédemment en relation avec le dispositif d'administration locale décrit.

Avantageusement, la formulation est sous la forme d'un cylindre de diamètre inférieur à 3 mm, de préférence inférieur à 1 mm et de longueur inférieure à 50 mm, de préférence inférieure à 30 mm, le volume total étant inférieur à 50 mm³, de préférence à 20 mm³.

L'invention a également pour objet une méthode de traitement thérapeutique comprenant l'injection à un patient nécessitant un traitement impliquant la libération d'un principe actif sur une période prolongée d'une formulation selon l'invention.

La formulation peut être injectée avantageusement par voie sous-cutanée ou intramusculaire.

Ceci peut être réalisé par tout moyen adapté, notamment une aiguille d'injection standard ayant un diamètre inférieur à 1 mm.

L'invention a également pour objet l'utilisation d'une formulation solide telle que définie ci-dessus pour l'obtention d'un effet retard.

Les exemples ci-après illustrent l'invention :

### EXEMPLE 1 :

### Insert intra-sinusien de Phosphate de Dexaméthasone, forme PLGA

La fabncation des inserts de Dexaméthasone phosphate se déroule selon les phases suivantes :
- Pesée des matières premières, mélange, première extrusion, broyage et tamisage, dosage et conditionnement, le tout sous flux laminaires classe A dans une salle blanche classe D, et enfin, stérilisation.

Pour un lot, on pourra, par exemple, peser 38,25 g de copolymère lactide-coglycolide PLGA (50:50) et incorporer 6,75 g de Dexaméthasone-21-phosphate disodique broyés à moins de 100 micromètres.

La poudre sera mélangée grâce au mélangeur à mouvement tridimensionnel et à la première extrusion, on contrôlera la qualité du mélange (% de PA).

Après extrusion, le mélange est broyé et extrudé à nouveau en joncs de 2 à 2,5 de diamètre dont on vérifie l'homogénéité (% PA, contenu PA/longueur). On calcule alors le poids de l'insert nécessaire à l'obtention d'une dose équivalente à 7,5 mg de Dexaméthasone phosphate. On coupe les cylindres aux longueurs correspondant au poids nécessaire et on les conditionne de façon individuelle dans les dispositifs- contenant qui seront gamma-irradiés (25 kGy).

Le dispositif pourra être directement utilisé à l'intérieur d'un trocart de 3 mm de diamètre et 10 cm de longueur selon les schémas des figures 5 à 10.

Avant de tester le sinus maxillaire, l'efficacité de ces inserts, par exemple, dans l'occlusion nasale chronique, on vérifie le relargage *in-vitro* et *in- vivo* sur un modèle susceptible d'être prédictif de la durée de l'insert.

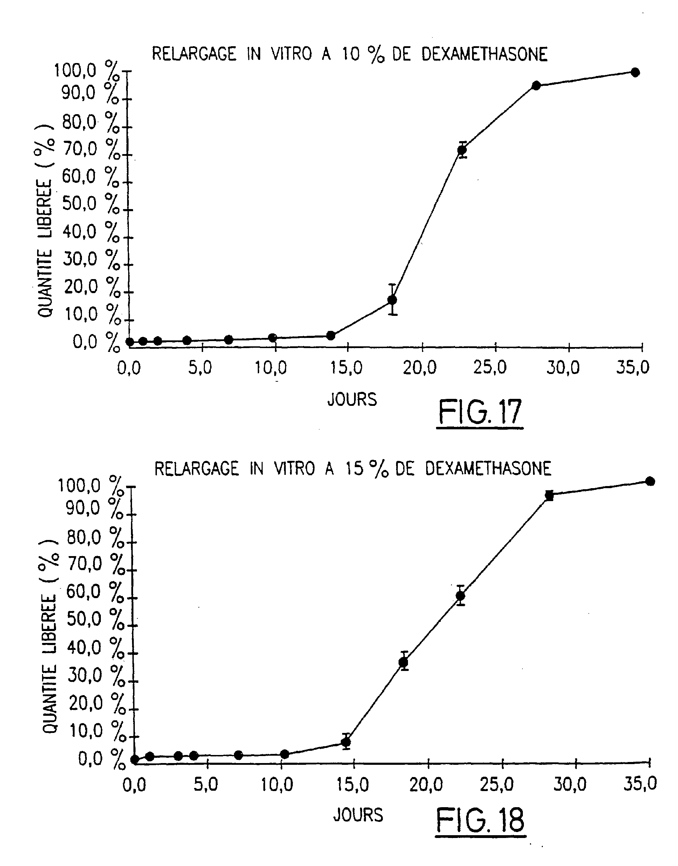

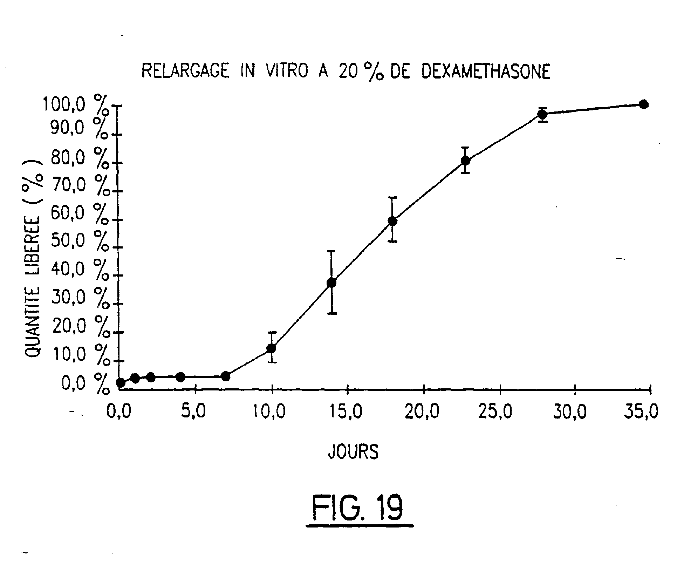

*In-vitro,* le relargage est suivi par dosage du PA en HPLC dans un milieu isotonique à l'intérieur duquel est plongé l'insert. Les figures 17, 18 et 19 représentent ces relargages *in-vitro* pour trois concentrations différentes de PA respectivement 10, 15 et 20 %.

*In-vivo,* un modèle sur le rat est utilisé. L'insert est administré soit en sous-cutané soit en intra-péritonéale et le relargage sur un mois est évalué par défaut en dosant la quantité de PA restant dans l'insert après sacrifice des animaux et prélèvement à des temps déterminés.

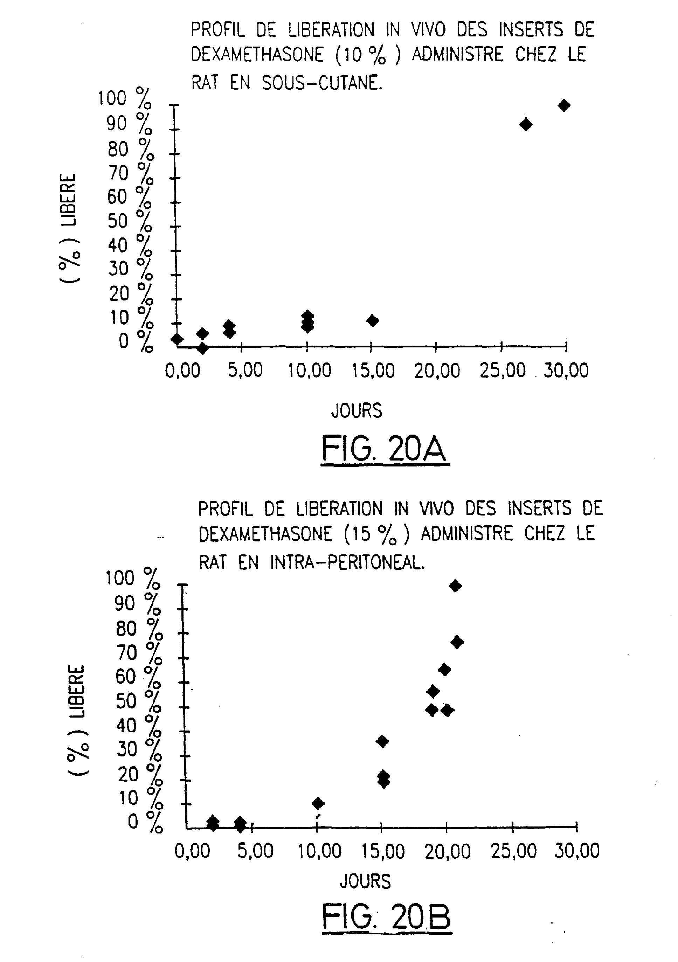

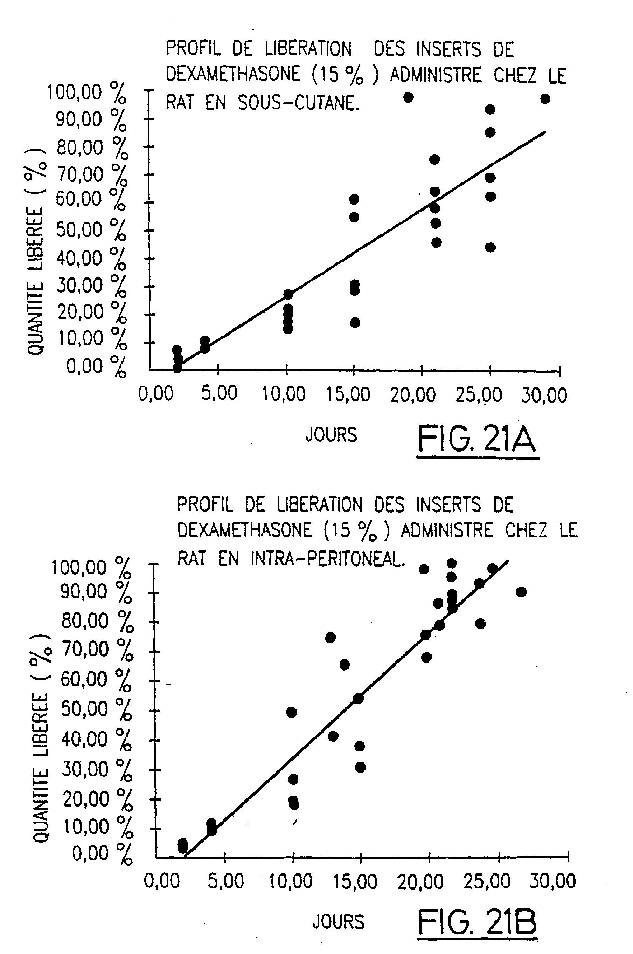

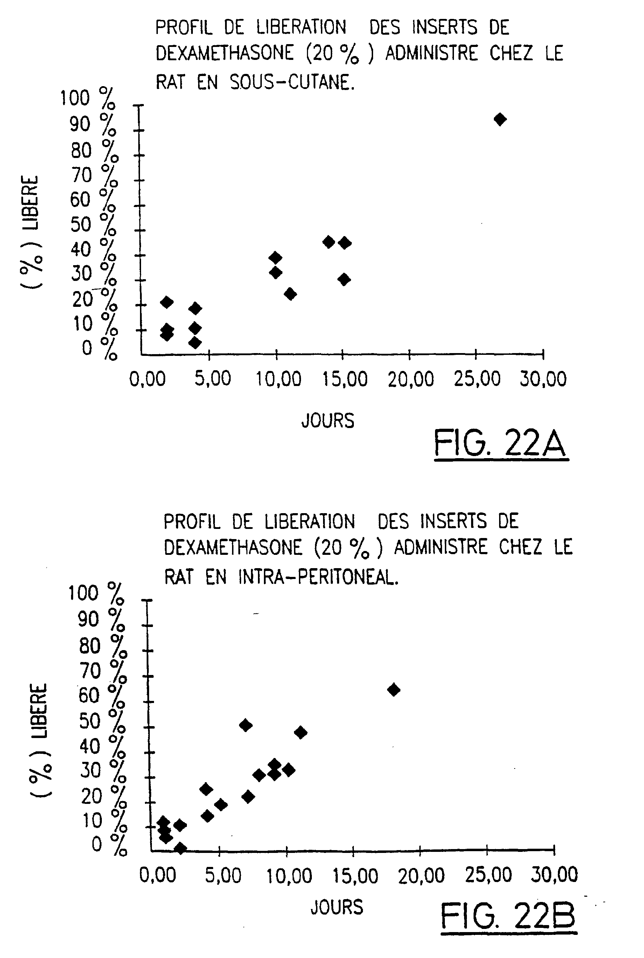

Les figures 20, 21 et 22 représentent les résultats de ce contrôle in-vivo aux trois pourcentages en sous-cutané (A) et en intra-péritonéale (B).

### EXEMPLE 2 :

### Implant transluminal d'Acétate de Lanréotide, forme solide

Des implants ou cylindres de 0,75 mm de diamètre et 30 mm de longueur ont été fabriqués. Ils contiennent 12,80 mg de Lanréotide (BIM2301-4C) pour une composition à 90 % d'Acétate de Lanréotide et 10 % de Mannitol.

Pour une taille de lot de 200 unités soit 4,5 g de solide (Acétate de Lanréotide-Mannitol), la fabrication comporte les étapes suivantes : pesée, connexion, vide, hydratation, mélange, extrusion, séchage, arrangement et irradiation.

La pesée correspond au volume de la solution eau-mannitol, d'une part, dans une seringue et à la poudre d'acétate de Somatuline dans l'autre seringue.

La connexion est l'association des deux seringues par l'intermédiaire d'une vanne boule 3 voies.

Le vide est alors réalisé à l'intérieur de la poudre de PA.

L'hydratation est obtenue en mettant en contact la poudre sous vide de la solution de Mannitol. Le mélange est réalisé par va et vient en actionnant les pistons des deux seringues. L'extrusion correspond après contrôle de l'homogénéité HPLC à la réalisation d'un jonc à travers une filière adaptée au diamètre souhaité. Cette extrusion est obtenue également en actionnant avec un moteur le piston de la seringue.

Le séchage est réalisé après ou avant la coupe des cylindres. Il consiste à évaporer l'eau du mélange pâteux pour obtenir le cylindre sec.

L'arrangement consiste à introduire le cylindre à l'intérieur de l'aiguille d'injection dans un dispositif de 1 mm de diamètre tel qu'il est représenté sur la figure 5.

L'irradiation par stérilisation, après emballage du dispositif, est réalisée avec 25 kGy.

Ce dispositif pourra être injecté au niveau local pour déposer le cylindre de Lanréotide avant ou après angiopiastie, comme un stent, par la lumière du cathéter.

L'effet retard local de cette formulation a été préalablement évalué en intra-muscuiaire (i.m.) sur le chien, et en i.m. et sous-cutané (s.c) chez l'homme.

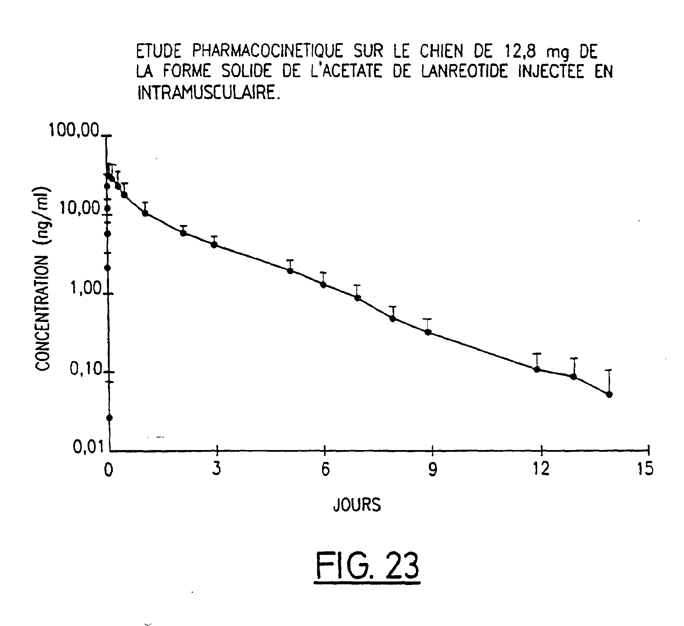

La figure 23 représente le résultat d'une pharmacocinétique sur le chien de la forme solide 12,8 mg de Lanréotide en intra-musculaire.

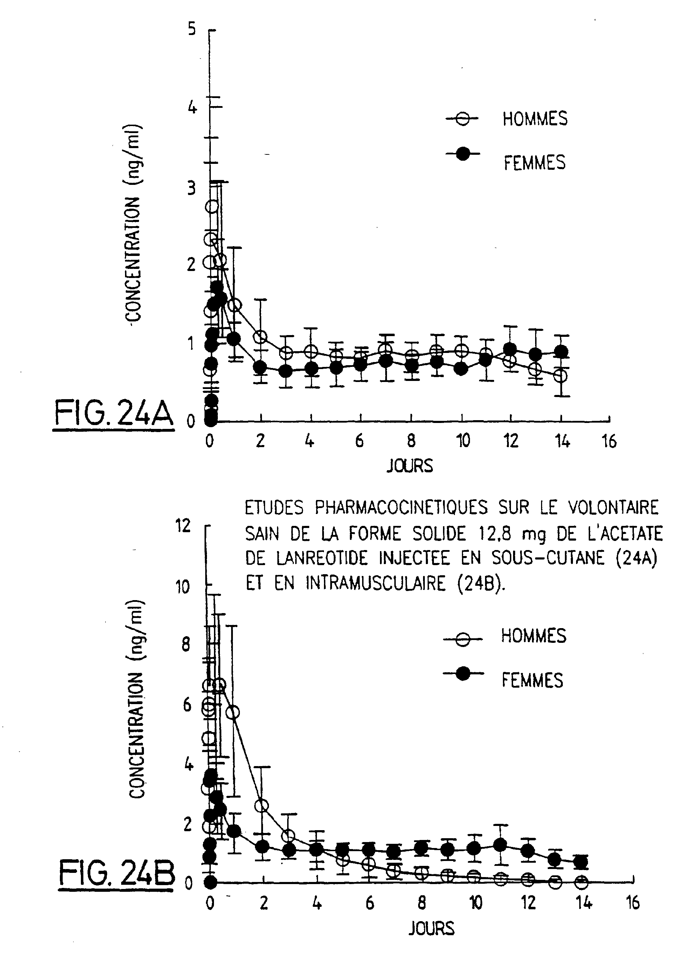

La figure 24 représente les résultats d'une cinétique chez le volontaire sain en sous-cutané (A) et en intra-muscuiaire (B).

Les résultats obtenus permettent d'envisager un effet retard prolongé au niveau local de l'angioplastie avec une concentration local élevée sur cette période.

### EXEMPLE 3 :

### Dépôt semi-solide d'Acétate de Lanréotide

L'acétate de Lanréotide forme avec de l'eau une pâte ou un semi-solide retard injectable.

L'effet retard est obtenu par dépôt, directement à partir du principe actif. Cet effet retard est modulable en fonction du pourcentage. La curée d'action est donc directement proportionnelle à l'érosion ou élimination de ce dépôt semi-solide. On peut donc associer tout autre principe actif dont on recherchera l'effet local combiné au Lanréotide. La durée d'action du ou des PA pourra être évaluée par la seule pharmacocinétique du Lanréotide.

On fabrique le semi-solide selon un procédé proche de celui du solide de l'exemple 2 sans Mannitol. L'extrusion, le séchage et le réarrangement sont remplacés par une distribution. Par exemple pour 200 unités, on préparera 40 g d'Acétate de Lanréotide dans le cas de la forme un mois 35 % acétate de Lanréotide, 65 % eau et pour des doses injectées de 40 mg de PA.

Les étapes de fabrication sont la pesée, la connexion, le vide, hydratation, le mélange, la distribution et irradiation.

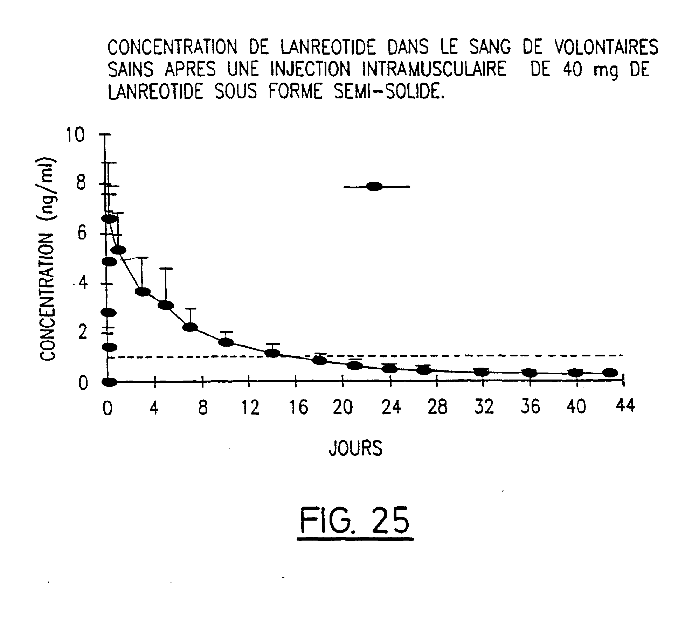

La distribution consiste en un remplissage volumétrique du dispositif d'injection (fig.11 à 16), par exemple, par piston rotatif à partir de la seringue de mélange. Cette formulation semi-solide a fait l'objet d'un essai clinique chez le volontaire sain en intra-musculaire (fig. 25).

On pourra donc obtenir ainsi une forme locale sur un mois. La concentration et la quantité de pâte détermineront la durée et l'intensité de la diffusion locale.

### EXEMPLE 4 :

### Comparaison d'une forme matricielle à 20 % de principe actif avec une forme non matricielle à 52 %.

On mélange un sel très soluble d'acétate de Triptoréline (AT) avec un PLGA (75:25) de plus de 100 000 de poids moléculaire et de viscosité inhérente égale à 1 dl/g dans le chloroforme qui ne subit une hydrolyse par perte de masse susceptible de contrôler une libération matricielle qu'après un mois.

On prépare ainsi des mélanges à 20 % (avant percolation) et à 52 % en poids de principe actif dans le PLGA. On extrude ces mélanges de façon à former des implants dont on vérifie le relargage *in vitro* à 37°C dans 10 ml de sérum physiologique et sans agitation.

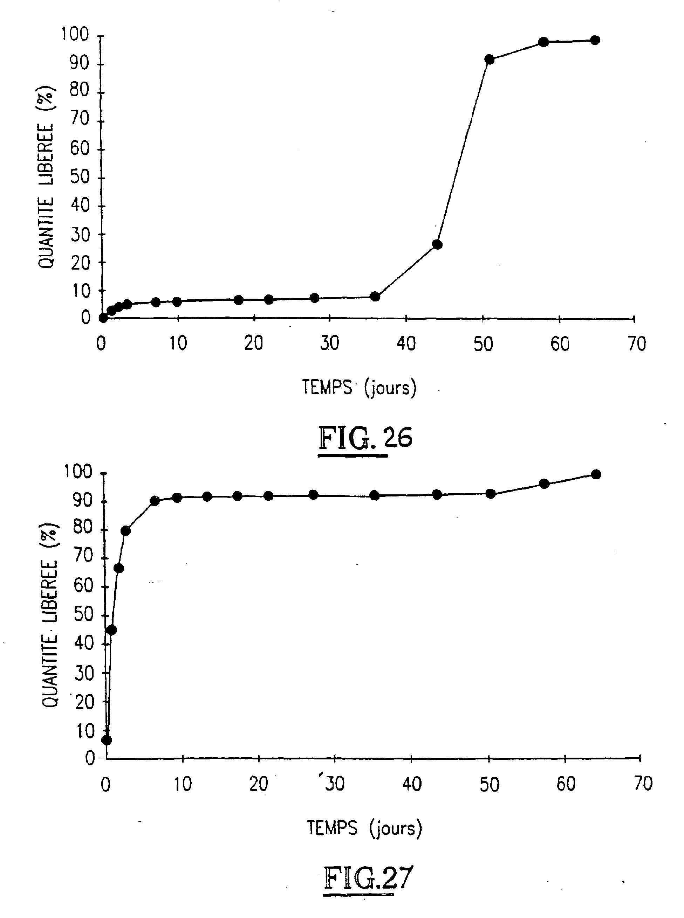

Les implants à 20 % de principe actif ne libèrent que 4 % de la dose totale en deux jours et seulement 6,7 % en 36 jours avant que ne s'opère la perte de masse du polymère qui entraîne la libération du principe actif entre J36 et J60 ( figure 26). Les implants à 52 % de principe actif libèrent 66 % de la dose totale en deux jours et plus de 90 % en une semaine (figure 27).

### EXEMPLE 5 :

### Comparaison d'une forme matricielle et non matricielle avec un sel insoluble de Triptoréline (pamoate de Triptoréline).

On prépare deux formulations de pamoate de Triptoréline et PLGA (50:50), la première à 40 % et la seconde à 52 % de principe actif.

On compare dans un modèle de reiargage *in vitro* la libération de ces deux formulations (la faible solubilité des principes actifs nécessite un volume de mise en suspension de 100 ml).

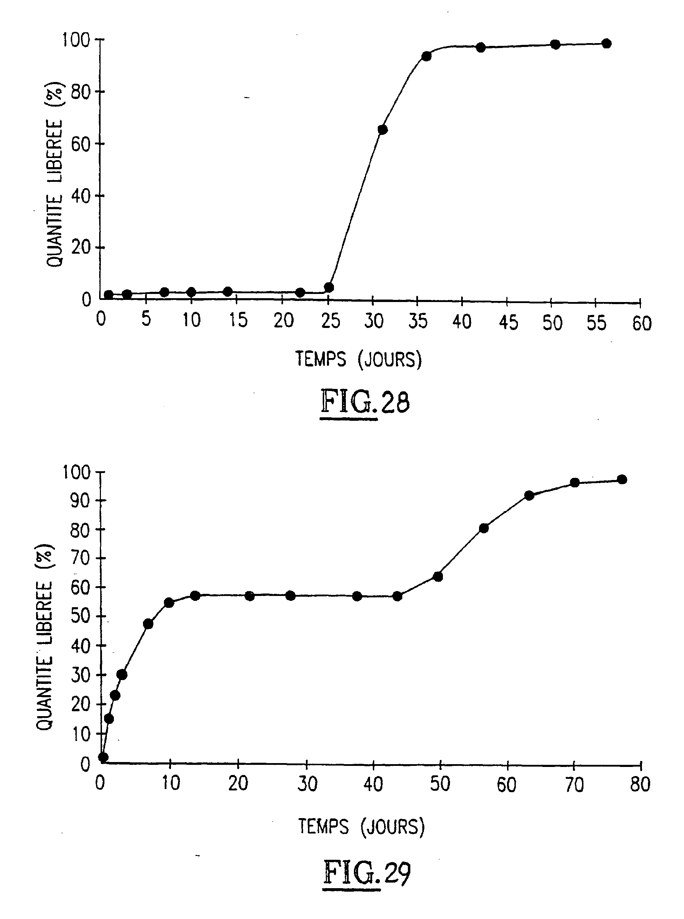

On observe, malgré l'insolubilité du principe actif un relargage de type matriciel avec 40 % (figure 28). A 52 % (figure 29), le relargage est déjà essentiellement indépendant de la matrice.

Le fonctionnement *in vitro* du principe actif par rapport au PLGA en mode matriciel et non matriciel ne dépend donc pas de la solubilité de son sel.

### EXEMPLE 6 :

### Différence macroscopique de mode d'action entre formulation matricielle et forme non matricielle

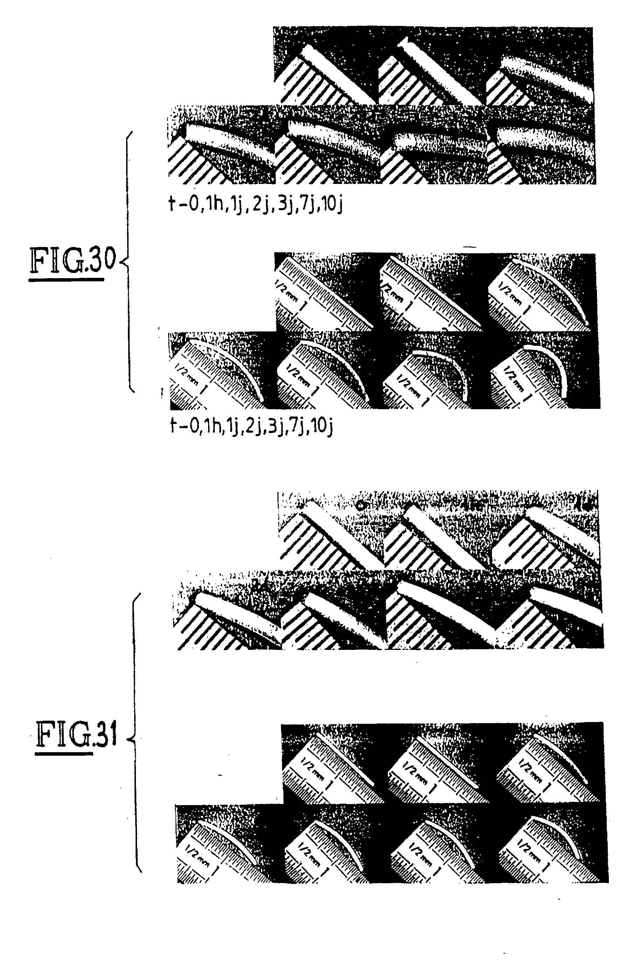

La préparation matricielle de l'exemple 4, PLGA 75:25 - acétate de Triptoréline (80 %-20 %) sous une forme non dispersée après dix jours dans un milieu physiologique *in vitro* contient pratiquement tout son principe actif ; elle présente un aspect translucide avec une augmentation de diamètre et une diminution de longueur par rapport au temps 0 (figure 30), ce qui démontre une contrainte de la matrice PLGA.

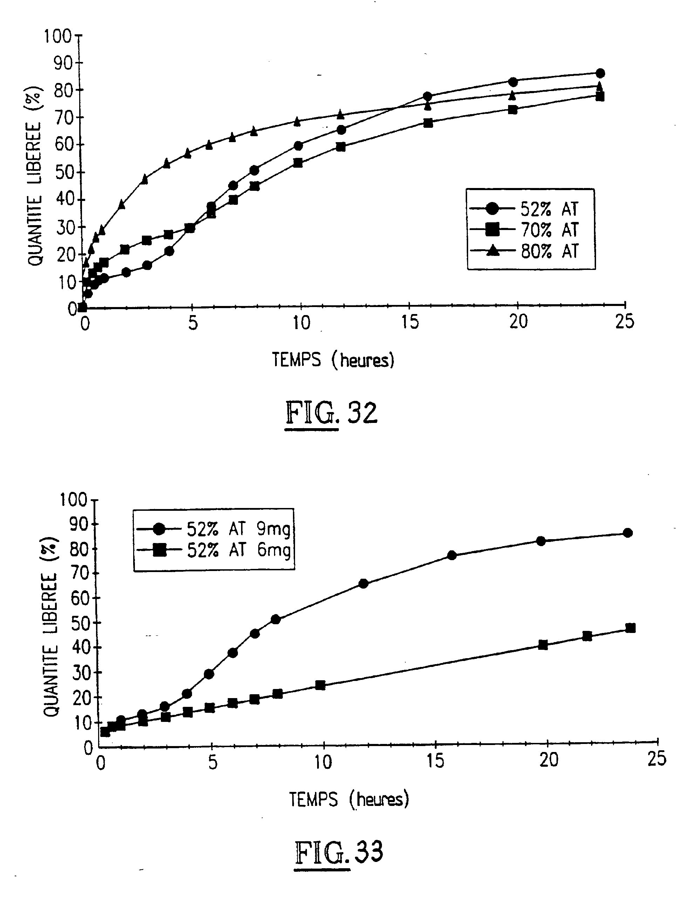

La préparation non matricielle PLGA 75:25 - Acétate de Triptoréline (48 %-52 %) dans les mêmes conditions après dix jours, est pratiquement totalement vidée de principe actif. Elle n'a pas subi de changement de diamètre ni de longueur (figure 31).

Le principe actif s'est donc échappé du squelette non matriciel de PLGA. Le principe actif est dans ce cas, libre de toute contrainte physico-chimique avec le polymère. Le PLGA reste inchangé au cours du relargage du principe actif.

### EXEMPLE 7 :

### Comparaison entre forme non matricielle (52 % d'acétate de Triptoréline) et formes non matricielles à 70 % et 80 % d'acétate de Triptoréline.

Dans le même modèle de reiargage *in vitro* qu'à l'exemple 4, on a comparé trois formes non matricielles à la même dose de 9 mg. Les résultats de relargage sur un jour (figure 32) démontrent une similitude de fonctionnement de ces trois formulations. La valeur de relargage atteinte *in vitro* n'est donc pas proportionnelle au C.L. Ceci démontre le rôle du principe actif et de sa quantité totale dans le fonctionnement des formes non matricielles.

### EXEMPLE 8:

### Comparaison de la libération in vitro des formes non matricielles à 52 % à la dose de 6 mg et 9 mg

Deux formulations utilisant le même PLGA 75:25 de PM supérieur à 100 000 ont été réalisées avec un C.L de 52 % d'acétate de Triptoréline (AT). Ces deux formulations ont été contrôlées *in vitro,* la première à la dose de 9 mg (52 % AT à 9 mg) et la seconde à la dose de 6 mg (52 % AT à 6 mg). Les résultats (figure 33) démontrent une différence de cinétique de relargage liée à la différence de dose de principe actif.

### EXEMPLE 9 :

### Comparaison des formes matricielles à 52 %, 70 % et 80 % de principe actif (acétate de Triptoréline) dans un essai in vivo chez le rat.

Deux lots d'implants à 52 % de principe actif, un lot d'implant à 70 % de principe actif et un lot d'implant à 80 % de principe actif ont été injectés en sous-cutané dans quatre groupes de 12 rats : 4 animaux de chaque groupe ont été sacrifiés à J1, J4 et J19. Les implants ont été récupérés et dosés en HPLC pour connaître les quantités résiduelles de principe actif.

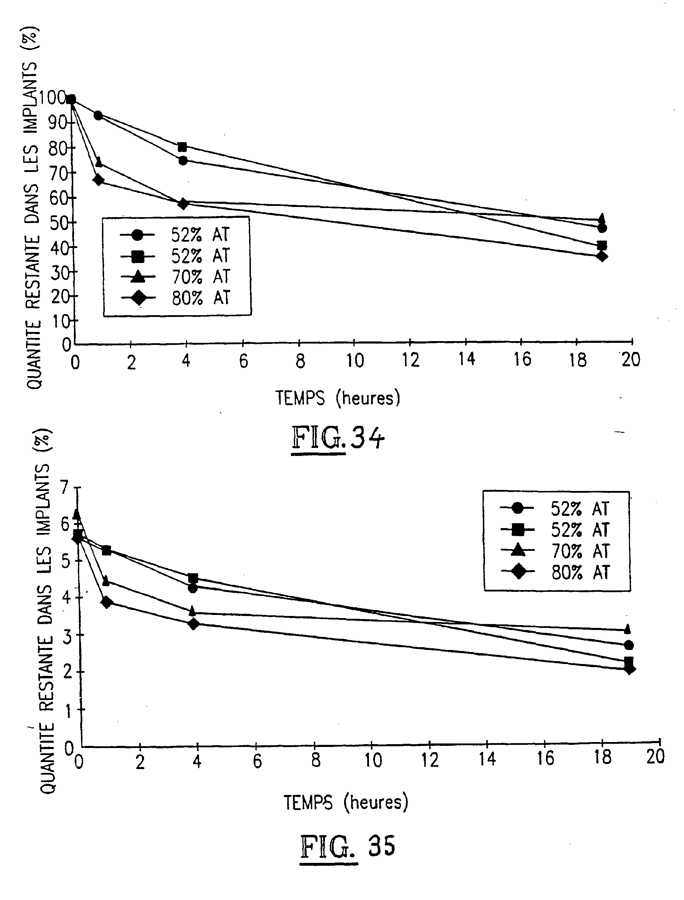

Les résultats de la figure 34 expriment entre J0 et J19 le taux résiduel des implants en pourcentage.

On note un parallélisme évident dans la diminution de ce pourcentage entre les formes à 52 %, à 70 % ou à 80 %.

La figure 35 représente l'évolution de la quantité résiduelle de principe actif pure en mg. On note que contrairement aux résultats *in vitro* après 19 jours, il reste en moyenne une quantité de principe actif importante et équivalente dans les implants à 52 % et dans les implants à 70 % et 80 %.

Des échantillons plasmatiques ont été prélevés sur ces animaux avant sacrifice et l'on a confirmé ce résultat par une analyse RIA.

### EXEMPLE 10 :

### Résultat pharmacocinétique d'une formulation matricielle (20 % de principe actif) et d'une formulation non matricielle (52 % de principe actif) sur le chien

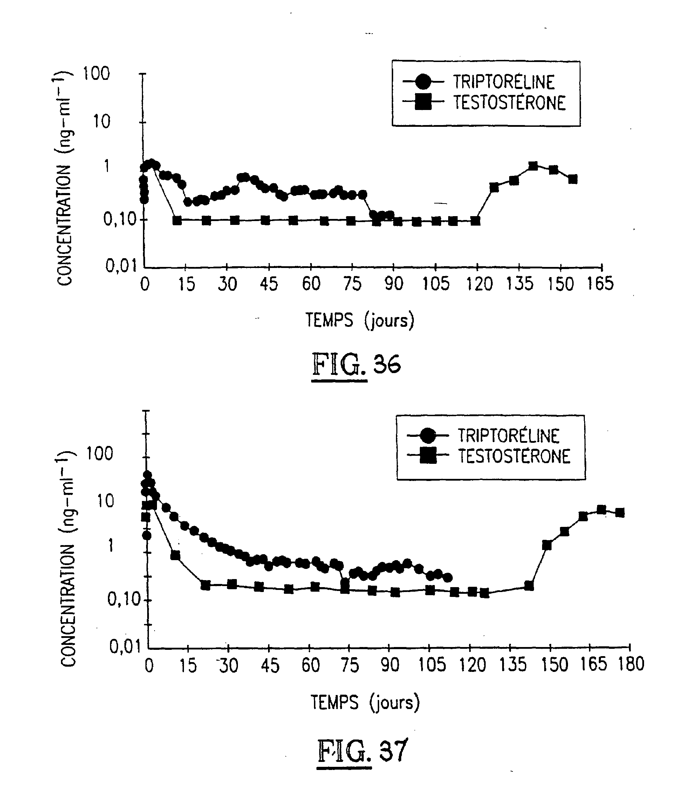

Les formulations à 20 % d'acétate de Triptoréline et à 52 % ont été injectées en IM en. deux séries à six chiens aux doses totales respectives de 3 et 6 mg de Triptoréline pure et on a suivi la cinétique par analyse RIA des échantillons piasmatiques ainsi que l'efficacité dynamique du principe actif avec les taux de testostérone (figures 36 et 37).

Les résultats démontrent une activité de relargage sur trois mois au moins dans les deux cas.

La cinétique de la forme à 20 % montre un profil classique (avec pic et rebond). La cinétique de la forme à 52 % n'est pas comparable à celles des formes PLGA classiques mais de pseudo ordre 0 sans pic ni rebond.

### EXEMPLE 11 : Résultats pharmacocinétiques d'une formulation non matricielle à 70 % de principe actif sur le chien.

Une formulation utilisant le même PLGA et le même principe actif que la formulation à 52 % de principe actif (exemple 10) a été réalisée avec 70 % et 30 % de PLGA.

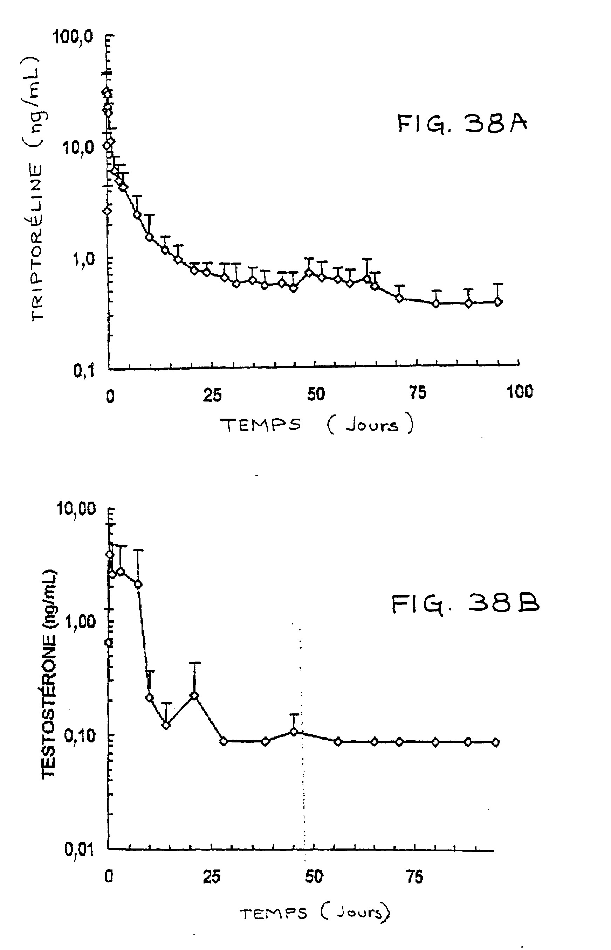

Cette formulation a été injectée en IM chez le chien à la dose totale de 9 mg de Triptoréline pure. On a suivi la cinétique par analyse RIA des échantillons plasmatiques (figure 38A) ainsi que l'efficacité dynamique du principe actif avec taux de testostérone (figure 38 B).

Les résultats montrent bien une activité de relargage sur au moins trois mois comme pour la forme à 52 % de principe actif avec comme seule différence un niveau de relargage plus élevé en faisant varier la dose totale.

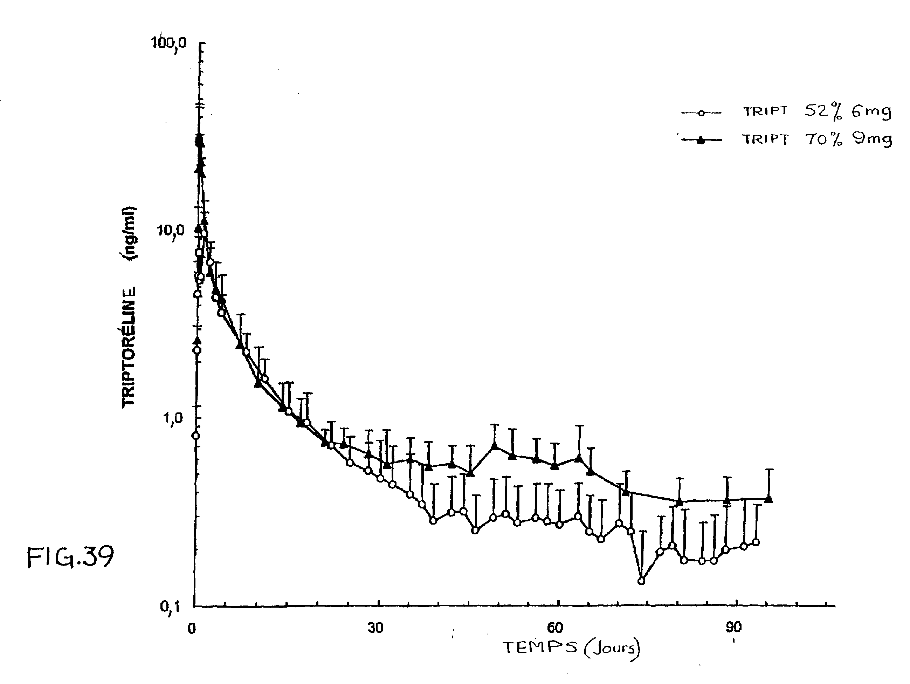

La variation de la charge entre 52 % et 70 % n'influence ni la durée ni le profil et le niveau de relargage dépend bien de la dose totale injectée (figure 39).

## Revendications

1. Formulation retard solide destinée à être mise en place dans un organisme, notamment par administration parentérale, comprenant un mélange homogène d'un principe actif à l'état non dispersé formant une phase continue, dont au moins une partie est en contact direct avec la surface d'échange de la formulation et du milieu biologique extérieur, et d'un excipient biocompatible biodégradable, dans laquelle la quantité de principe actif est d'au moins 50% en poids par rapport au poids total de la formulation, et ayant un profil de relargage indépendant de la composition de l'excipient, du poids moléculaire de l'excipient ou du rapport pondéral principe actif/excipient, le profil de relargage étant essentiellement exclusivement dépendant de la quantité totale de principe actif présent dans la formulation.

2. Formulation retard solide, destinée à être mise en place dans un organisme, contenant au moins un principe actif et un excipient biodégradable, **caractérisée en ce que** l'excipient biodégradable, **caractérisée en ce que** l'excipient est un copolymère polylactide-glycolide (PLGA), et **en ce que** la concentration de principe actif est comprise entre 10 et 100%.

3. formulation retard selon la revendication 2, **caractérisée en ce que** la concentration de principe actif est comprise entre 50 et 100%.

4. Formulation retard selon l'une des revendications 1 à 3, destinée à être mise en place en un site de dépôt précis d'un organisme, contenant au moins un principe actif, ladite formulation étant de consistance solide ou semi-solide telle qu'elle puisse persister pendant une certaine durée dans le site, et contenant une dose de principe actif limitée pour un traitement dans une zone ciblée de l'organisme.

5. Formulation selon l'une des revendications 1 à 4, **caractérisée en ce qu'**elle contient une dose de principe actif basse par rapport à la dose habituelle pour un traitement par voie systémique du principe actif considéré.

6. Formulation selon l'une des revendications 1 à 5, **caractérisée en ce qu'**elle possède une forme fine et allongée, notamment cylindrique.

7. Formulation selon la revendication 6, **caractérisée en ce qu'**elle présente un diamètre compris entre 0,1 et 2 à 3 mm.

8. Formulation retard selon la revendication 7, **caractérisée par** un diamètre ne dépassant pas 2 mm.

9. Formulation retard selon la revendication 7, **caractérisée par** un diamètre de l'ordre de 0,1 mm.

10. Formulation selon l'une des revendications 6 à 9, **caractérisée en ce que** la forme présente un rapport minimum longueur/diamètre de 10.

11. Formulation selon l'une des revendications 1 à 10, de nature solide, susceptible d'être déformée en étant précontrainte dans un dispositif d'administration pour retrouver sa forme in situ.

12. Formulation selon l'une des revendications 1 et 4 à 11, **caractérisée en ce que** l'excipient biocompatible biodégradable est un polymère ou copolymère d'acide lactique et/ou glycolique ou un mélange de polymères et/ou copolymères d'acide lactique et/ou glycolique.

13. Formulation selon la revendication 12, **caractérisée en ce que** ledit polymère biocompatible biodégradable est un copolymère d'acide lactique et d'acide glycolique (PLGA).

14. Formulation selon l'une des revendications 1 à 13, **caractérisée en ce que** ledit polymère biocompatible biodégradable est un copolymère d'acide lactique et glycolique ayant une viscosité intrinsèque dans le chloroforme à 1 lg pour 100 ml supérieure à 0,6 dl/g.

15. Formulation selon l'une des revendications 1 à 14, **caractérisée en ce que** le copolymère d'acide lactique et d'acide glycolique est de nature hydrophile.

16. Formulation selon l'une des revendications 1 à 15, **caractérisée en ce que**, lorsqu'elle est placée in vitro dans un milieu liquide physiologique, elle libère la quasi-totalité du principe actif en moins d'une semaine, et, lorsqu'elle est placée *in vivo* en sous-cutané ou en intra-musculaire, présente un relargage du principe actif sur une période substantiellement supérieure à une semaine.

17. Formulation selon l'une des revendications 1 à 16, **caractérisée en ce qu'**elle comprend un mélange en tous points homogène du principe actif et de l'excipient.

18. Formulation selon l'une des revendications 1 à 17, **caractérisée en ce que** le relargage s'effectue en une seule phase de diffusion du principe actif.

19. Formulation selon l'une des revendications 1 à 18, **caractérisée en ce que** le principe actif représente au moins 51% et jusqu'à 99,999% en poids par rapport au poids total de la formulation, l'excipient représentant moins de 50%, de préférence moins de 49%, et plus avantageusement moins de 30% en poids par rapport au poids total de la formulation.

20. Formulation selon l'une des revendications 1 à 19, **caractérisée en ce que** le principe actif est un peptide, un analogue peptidique ou une protéine, notamment la LHRH ou un analogue de la LHRH, notamment la Triptoréline.

21. Formulation selon l'une des revendications 1 à 20 pour injection par voie intra-musculaire ou sous-cutanée.

22. Formulation retard selon l'une des revendications 1 à 20, **caractérisée en ce qu'**elle est sous la forme d'un implant.

23. Formulation selon l'une des revendications 1 à 22, **caractérisée en ce qu'**elle est agencée pour que le relargage du principe actif ait lieu dans une cavité anatomique dans laquelle elle a été introduite.

24. Formulation selon la revendication 23, **caractérisée en ce qu'**elle a une forme étudiée pour pouvoir être emprisonnée dans une cavité anatomique de l'organisme en évitant le déplacement ou l'élimination de la formulation.

25. Formulation selon la revendication 24, dans laquelle la longueur et le diamètre de la formulation sont agencés pour éviter son élimination ou son déplacement.

26. Formulation selon l'une des revendications 1 à 25, **caractérisée en ce que** ladite formulation et le principe actif qu'elle contient sont agencés pour que le relargage du principe actif ait lieu dans les sécrétions d'une muqueuse.

27. Formulation selon l'une des revendications 23 à 26, dans laquelle ladite cavité ou muqueuse est une cavité ou muqueuse de la sphère faciale ou ORL.

28. Formulation selon la revendication 26, dans laquelle ladite muqueuse est une muqueuse trachéo-pulmonaire.

29. Formulation selon la revendication 26, dans laquelle ladite muqueuse est la muqueuse buco-oesophagienne.

30. Formulation selon l'une des revendications 26 à 29, dans laquelle ladite formulation est agencée pour être placée à la surface de ladite muqueuse de façon que le principe actif soit véhiculé par le mucus.

31. Formulation selon l'une des revendications 26 à 30, dans laquelle ladite formulation est agencée pour être placée à l'intérieur de la muqueuse.

32. Formulation selon la revendication 31, **caractérisée en ce que** ladite formulation et le principe actif qu'elle contient sont agencés pour une injection dans la muqueuse sous-palpébrale.

33. Formulation selon l'une des revendications 23 à 32, **caractérisée en ce qu'**elle comprend un corticoïde adapté au traitement, dans une cavité, paroi de cavité ou muqueuse, de la polypose naso-sinusienne, des rhinites allergiques ou non, des otites ou sinusites non infectieuses, par introduction dans le sinus maxillaire, phénoïdal ou frontal, la muqueuse nasale, les cellules ethmoïdales ou la caisse du tympan.

34. Formulation selon l'une des revendications 1 à 22, **caractérisée en ce que** ladite formulation et le principe actif qu'elle contient sont agencés pour une introduction dans ou autour de la paroi vasculaire par injection intra ou trans-luminale.

35. Formulation selon la revendication 34, utilisable notamment après angioplastie percutanée transluminale, comprenant un principe actif pour la prévention ou le traitement de la resténose.

36. Formulation selon la revendication 35, contenant de l'angiopeptine seule ou associée à un autre principe actif, notamment l'héparine.

37. Formulation selon l'une des revendications 1 à 22, **caractérisée en ce que** ladite formulation et le principe actif qu'elle contient sont agencés pour être introduits dans ou sous un tissu tumoral pour une action antitumorale.

38. Formulation selon la revendication 37, dans laquelle le principe actif comprend un produit photosensible.

39. Formulation selon l'une des revendications 1 à 22, **caractérisée en ce que** ladite formulation et le principe actif qu'elle contient sont agencés pour une injection intra ou péri-articulaire.

40. Formulation selon l'une des revendications 1 à 39, **caractérisée en ce qu'**elle contient un principe actif anti-inflammatoire.

41. Utilisation d'une formulation retard selon l'une des revendications 1 à 40 pour la préparation d'un médicament destiné à une injection parentérale sous forme sèche.

42. Procédé de préparation d'une formulation retard selon l'une des revendications 1 à 40, comportant les étapes consistant à :
- réaliser un mélange homogène du principe actif et de l'excipient, contenant au moins 50% de principe actif ;
- extruder ledit mélange compacté à l'état fondu.

43. Procédé de préparation d'une formulation selon l'une des revendications 1 à 40, comportant les étapes consistant à :
- réaliser un mélange homogène du principe actif et de l'excipient, contenant au moins 50% de principe actif ;
- soumettre le mélange homogène à une compression élevée ;
- broyer les comprimés obtenus ; et
- mettre sous une forme adaptée à l'administration.
